# EUROPEAN PATENT SPECIFICATION

(11) **EP 3 243 447 B1**
(45) Date of publication and mention of the grant of the patent: **29.04.2020**
(21) Application number: 17169869.9
(22) Date of filing: 08.05.2017
(51) Int. Cl.: A61B 17/072

(54) **ADAPTER ASSEMBLY FOR SURGICAL STAPLING DEVICES**
ADAPTERANORDNUNG FÜR CHIRURGISCHE KLAMMERVORRICHTUNGEN
ENSEMBLES ADAPTATEUR POUR DISPOSITIFS D'AGRAFAGE CHIRURGICAL

(30) Priority: 09.05.2016 US 201615149839
(43) Date of publication of application: 15.11.2017
(73) Proprietor: Covidien LP, Mansfield, MA 02048 (US)
(72) Inventor: BEARDSLEY, John, Wallingford, CT 06492 (US); PRIBANIC, Russell, Roxbury, CT 06783 (US); NICHOLAS, David, Trumbull, CT 06611 (US); MARCZYK, Stanislaw, Stratford, CT 06614 (US)
(74) Representative: Maschio, Antonio

(56) References cited:
- EP-A1- 2 505 146
- EP-A2- 2 687 164
- WO-A1-98/14124
- WO-A1-2006/048905
- US-A1- 2011 163 146
- US-A1- 2014 005 678
- US-A1- 2014 367 447

## Description

### CROSS-REFERENCE TO RELATED APPLICATION

### BACKGROUND

### 1. Technical Field

The present disclosure relates to surgical stapling devices. More specifically, the present disclosure relates to adapter assemblies for use with the powered surgical stapling devices and loading units for use with the adapter assemblies.

### 2. Background of Related Art

Surgical stapling devices having a powered handle are known. Such devices typically include an adapter assembly for connecting loading units to the powered handle. Often the adapter assemblies transfer rotational motion from one or more motors in the powered handle assembly into linear motion to actuate and articulate the attached loading units. Because replacing a used disposable loading unit or replacing a used cartridge assembly of a multiple use loading unit during a surgical procedure may be a complicated and/or time consuming process, it would be desirable to have an adapter assembly and loading unit with an easily attachable and replaceable loading unit. It would be further desirable to have an adapter assembly with a flexible shaft for increasing the ability to maneuver the loading unit to a desired location.

Document EP2687164 discloses an adapter assembly that includes a rigid portion at its proximal end interconnecting a drive coupling assembly and a flexible portion or flexible shaft. The shaft assembly also includes a pivoting linkage interconnecting the flexible portion and an end effector.

Document US2011163146 discloses an adapter assembly wherein its elongate shaft is hollow and is formed from multiple connecting segments to allow the elongate shaft to flex. In order to allow movement of an end effector relative to the elongate shaft, the end effector can be movably coupled to the distal end of the elongate shaft. For example, the end effector can be pivotally coupled to the distal end of the elongate shaft by a pivoting or rotating joint.

### SUMMARY

An adapter assembly for selectively interconnecting a surgical end effector that is configured to perform a function and a surgical device that is configured to actuate the end effector is provided. The end effector includes at least one axially translatable drive member, and the surgical device includes at least one rotatable drive shaft. The adapter assembly includes a proximal body portion configured for operable connection to an actuation assembly, and a distal body portion selectively articulable relative to the proximal body portion at an articulation joint. The distal body portion includes a distal end configured for selective engagement with a loading unit and defines a longitudinal axis. A length of the proximal body portion may be flexible.

The proximal body portion may include a handle portion and includes a flexible portion. The adapter assembly may include a first elongate rigid portion disposed between the handle portion and the flexible portion. The adapter assembly may further include a second elongate rigid portion extending distally from the flexible portion. The proximal body portion defines a working channel therethrough. The working channel includes a proximal opening adjacent a proximal end of the proximal body portion and a distal opening adjacent a distal end of the proximal body portion. The adapter assembly may include a loading unit selectively engageable with the distal end of the distal body portion. The adapter assembly may include an articulation assembly disposed within the proximal body portion. The handle portion may include a frustoconical body.

Another adapter assembly for selectively interconnecting a surgical end effector that is configured to perform a function and a surgical device that is configured to actuate the end effector is provided. The end effector includes at least one axially translatable drive member, and the surgical device includes at least one rotatable drive shaft. The adapter assembly includes a proximal body portion configured for operable connection to an actuation assembly, the proximal body portion defining a working channel and a distal body portion selectively articulable relative to the proximal body portion at an articulation joint. The distal body portion includes a distal end configured for selective engagement with a loading unit and defines a longitudinal axis.

The proximal body portion may include a handle portion and a flexible portion. The adapter assembly may include a first elongate rigid portion disposed between the handle portion and the flexible portion. The adapter assembly may further include a second elongate rigid portion extending distally from the flexible portion. The working channel includes a proximal opening adjacent a proximal end of the proximal body portion and a distal opening adjacent a distal end of the proximal body portion. The adapter assembly may include a loading unit selectively engageable with the distal end of the distal body portion. The adapter assembly may include an articulation assembly disposed within the proximal body portion. The handle portion may include a frustoconical body.

### BRIEF DESCRIPTION OF THE DRAWINGS

Embodiments of the present disclosure are described herein with reference to the accompanying drawings, wherein:
FIG. 1 is a perspective view of an electromechanical surgical stapling device including an adapter assembly, loading unit, and cartridge assembly, in accordance with an embodiment of the present disclosure, and a powered handle assembly;
FIG. 2 is a perspective view of the powered handle assembly shown in FIG. 1;
FIG. 3 is a perspective side view of the adapter assembly shown in FIG. 1;
FIG. 4 is a cross-sectional side view of the adapter assembly and the loading unit shown in FIG. 1;
FIG. 5 is an enlarged view of the indicated area of detail shown in FIG. 4;
FIG. 6 is a cross-sectional top view of the adapter assembly, the loading unit, and cartridge assembly shown in FIG. 1;
FIG. 7 is an enlarged view of the indicated area of detail shown in FIG. 6;
FIG. 8 is a perspective end view of the adapter assembly shown in FIG. 1 with an outer sleeve removed and a proximal end shown in phantom;
FIG. 9 is an enlarged view of the indicated area of detail shown in FIG. 8;
FIG. 10 is an exploded perspective view of a proximal body portion of the adapter assembly shown in FIG. 1;
FIG. 11 is an exploded perspective view of an articulation assembly of the adapter assembly shown in FIG. 1;
FIG. 12 is a cross-sectional top view of a distal end of the adapter assembly and the loading unit shown in FIG. 1;
FIG. 13 is a perspective top view of the distal end of the adapter assembly and a proximal end of the loading unit shown in FIG. 1, with connector housings and the outer sleeve of the adapter assembly shown in phantom;
FIG. 14 is a perspective side view of the articulation assembly shown in FIG. 11;
FIG. 15 is an exploded perspective view of a distal body portion of the adapter assembly shown in FIG. 1;
FIG. 16 is an enlarged perspective view of the distal end of the adapter assembly and an actuation assembly of the loading unit shown in FIG. 1, with housings shown in phantom;
FIG. 17 is an enlarged perspective view of the distal end of the adapter assembly shown in FIG. 1 with the housings shown in phantom;
FIG. 18 is a cross-sectional end view taken along line 18-18 in FIG. 16;
FIG. 19 is a perspective side view of the loading unit and the cartridge assembly shown in FIG. 1;
FIG. 20 is an exploded perspective view of the loading unit shown in FIG. 1;
FIG. 21 is an enlarge perspective side view of the distal end of the adapter assembly and the proximal end of the loading unit shown in FIG. 1, as the loading unit is secured to the adapter assembly;
FIG. 22 is a perspective side view of the distal end of the adapter assembly and the proximal end of the loading unit shown in FIG. 21, with the loading unit secured to the adapter assembly;
FIG. 23 is an enlarged view of the indicated area of detail shown in FIG. 4;
FIG. 24 is a perspective side view of a drive beam of the loading unit shown in FIG. 1;
FIG. 25 is a cross-sectional side view of the drive beam shown in FIG. 24;
FIG. 26 is a cross-sectional perspective end view of the drive beam shown in FIG. 24;
FIG. 27 is a perspective side view of the distal end of the adapter assembly and a proximal end of the actuation assembly shown in FIG. 16, further including a locking mechanism in a first or distal position;
FIG. 28 is a perspective view of the distal end of the adapter assembly, the proximal end of the actuation assembly, and the locking mechanism shown in FIG. 27, further including a proximal end of the cartridge assembly shown in FIG. 1, with the locking mechanism in the second or proximal position;
FIG. 29 is an enlarged view of the indicated area of detail shown in FIG. 12;
FIG. 30 is a perspective end view of the cartridge assembly shown in FIG. 1;
FIG. 31 is an enlarged view of the indicated area of detail shown in FIG. 30;
FIG. 32 is a perspective side view of an alternative embodiment of a distal end of an adapter assembly and loading unit according to the present disclosure;
FIG. 33 is an exploded perspective side view of the proximal end of the adapter assembly shown in FIG. 32;
FIG. 34 is a perspective side view of the loading unit shown in FIG. 32;
FIG. 35A is a perspective end view of a plunger member of the loading unit shown in FIG. 32,
FIG. 35B is another perspective end view of the plunger member shown in FIG. 35A;
FIG. 36 is a cross-sectional top view of the loading unit shown in FIG. 32;
FIG. 37 is an enlarged view of the indicated area of detail shown in FIG. 32 with the anvil assembly in a closed position;
FIG. 38 is an enlarged view of the indicated area of detail shown in FIG. 36, with the anvil assembly in the closed position;
FIG. 39 is an enlarged view of the indicated area of detail shown in FIG. 32 with the anvil assembly in an opened position;
FIG. 40 is an enlarged view of the indicated area of detail shown in FIG. 36, with the anvil assembly in the opened position;
FIG. 41 is a perspective view of an electromechanical surgical stapling device including an adapter assembly, loading unit, and cartridge assembly, in accordance with an embodiment according to the present invention, secured to a powered handle assembly and including an instrument received through a working channel of the adapter assembly;
FIG. 42 is a perspective view of the adapter assembly shown in FIG. 41;
FIG. 43 is a perspective view of the adapter assembly shown in FIG. 41 in a flexed condition;
FIG. 44 is a cross-sectional top view taken along line 44-44 shown in FIG.42;
FIG. 45 is an enlarged view of the indicated area of detail shown in FIG. 44; and
FIG. 46 is a perspective top view of a proximal body portion of the adapter assembly shown in FIG. 41 with a handle portion split in half and each of a housing of a coupling assembly and a working channel of the adapter assembly shown in phantom.

### DETAILED DESCRIPTION OF EMBODIMENTS

Embodiments of the presently disclosed adapter assembly and loading units for surgical devices and/or handle assemblies are described in detail with reference to the drawings, in which like reference numerals designate identical or corresponding elements in each of the several views. As used herein the term "distal" refers to that portion of the adapter assembly or surgical device, or component thereof, farther from the user, while the term "proximal" refers to that portion of the adapter assembly or surgical device, or component thereof, closer to the user.

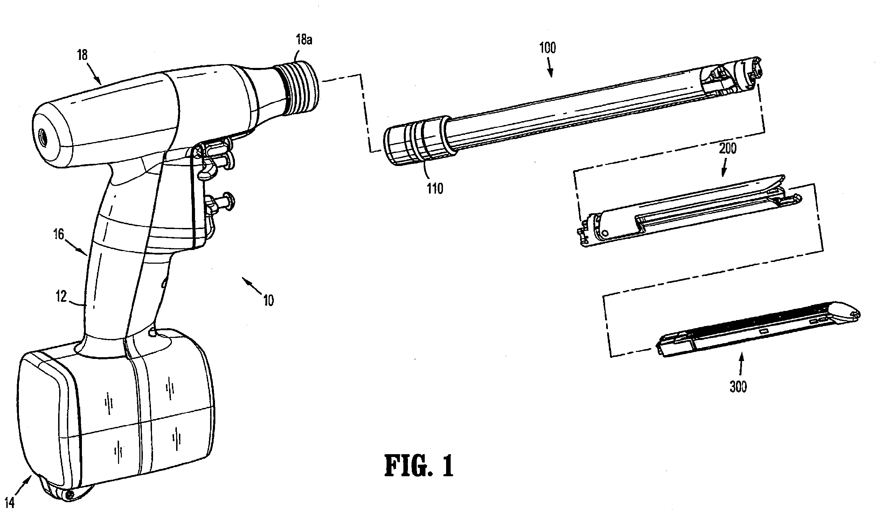

With reference to FIG. 1, an adapter assembly in accordance with an embodiment of the present disclosure, shown generally as adapter assembly 100, and a loading unit according to an embodiment of the present disclosure, shown generally as loading unit 200, are configured for selective connection to a powered hand-held electromechanical instrument, shown generally as handle assembly 10. The adapter assembly 100 is configured for selective connection with the handle assembly 10, and, the loading unit 200 is configured for selective connection with the adapter assembly 100. As will be shown and described in detail below, the loading unit 200 is a multiple use loading unit ("MULU") configured to selectively receive a replaceable loading unit, i.e., cartridge assembly 300. The adapter assembly 100, the loading unit 200 and the cartridge assembly 300 operate to staple and cut tissue. Although shown and described as being formed independent of the handle assembly 10, it is envisioned that the aspects of the adapter assembly 100 may be directly incorporated into the handle assembly 10.

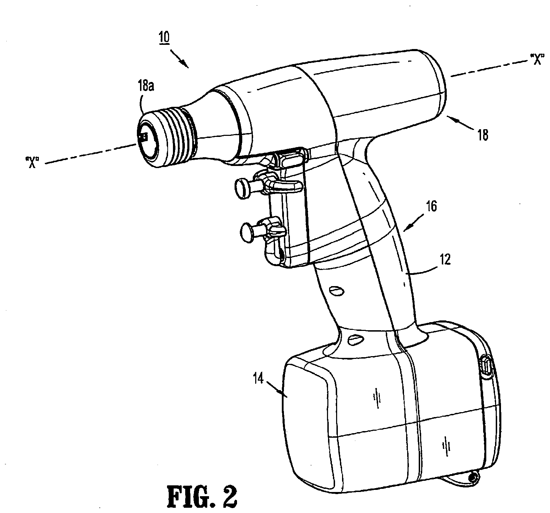

As illustrated in FIGS. 1 and 2, the handle assembly 10 includes a handle housing 12 having a lower housing portion 14, an intermediate housing portion 16 extending from and/or supported on the lower housing portion 14, and an upper housing portion 18 extending from and/or supported on the intermediate housing portion 16. A distal half-section of the upper housing portion 18 defines a nose or connecting portion 18a configured to accept a corresponding drive coupling assembly 110 of the adapter assembly 100. For a detailed description of the structure and function of an exemplary powered hand-held electromechanical instrument, please refer to commonly owned U.S. Pat. Appl. Publ. No. 2012/0253329 ("the '329 application") and U.S. Pat. Appl. Publ. No. 2012/0323226 ("the '226 application).

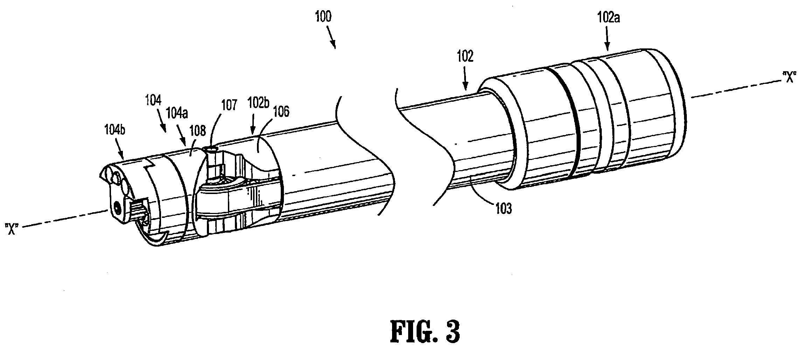

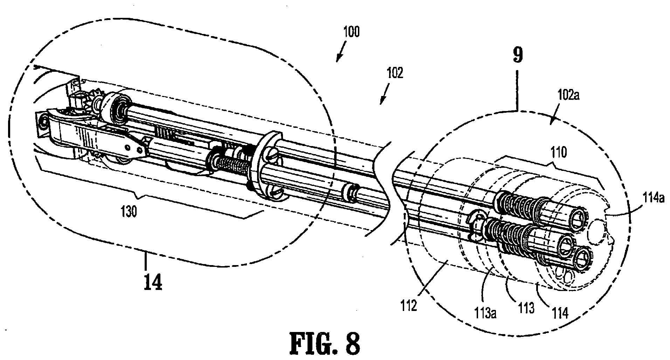

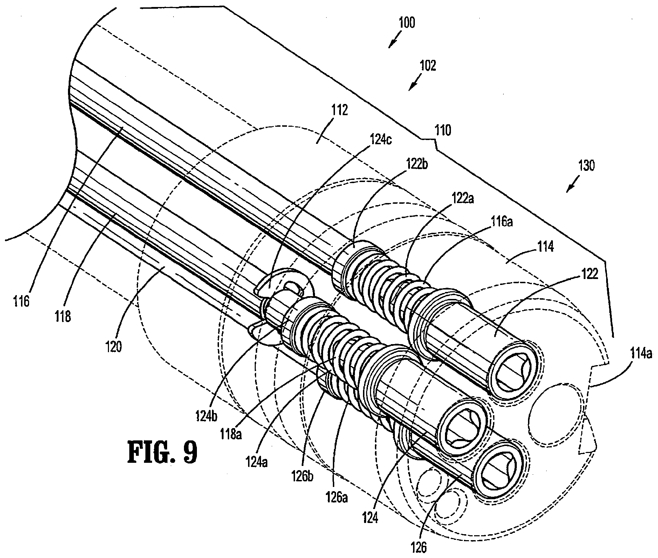

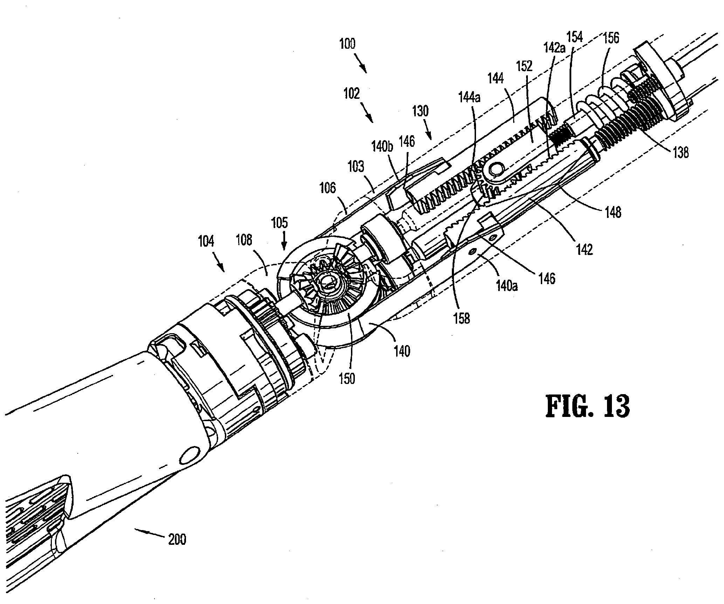

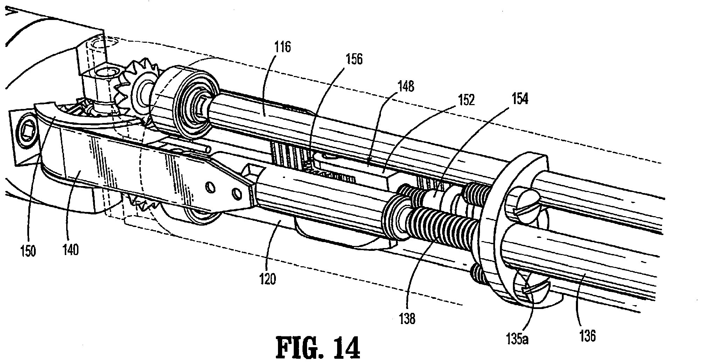

The adapter assembly 100 will now be described with reference to FIGS. 3-18. Referring initially to FIG. 3, the adapter assembly 100 includes a proximal body portion 102 and a distal body portion 104. The proximal body portion 102 includes a proximal end 102a configured for operable connection to the connecting portion 18a (FIG. 2) of the handle assembly 10 (FIG. 2) and a distal end 102b pivotally connected to a proximal end 104a of the distal body portion 104 at an articulation joint 105 (FIG. 3). More particularly, and as will be described in further detail below, a first connector housing 106 is disposed on the distal end 102b of the proximal body portion 102 and is pivotally secured to a distal connector housing 108 disposed on the proximal end 104a of the distal body portion 104 at the articulation joint 105 by a pivot pin 107. The distal body portion 104 includes a distal end 104b configured for selective connection to the loading unit 200 (FIG. 1). As will also be described in further detail below, the compact length of the distal body portion 104 allows for greater manipulation of an attached loading unit 200 within a body cavity (not shown) during a surgical procedure. The ability of the distal body portion 104 to pivot through an arc of one-hundred eight degrees (180°) relative to the proximal body portion 102 allows for further manipulation of an attached loading unit 200.

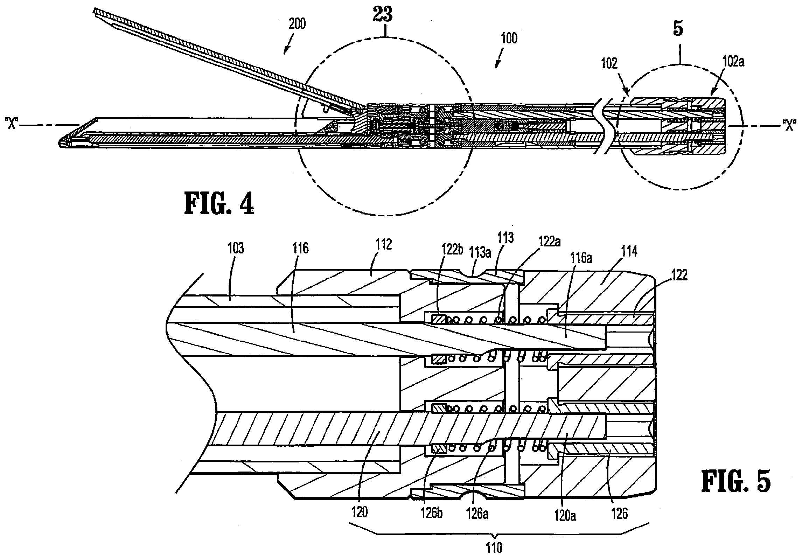

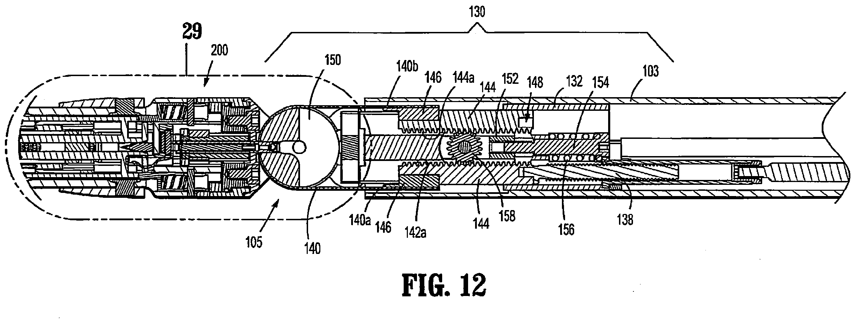

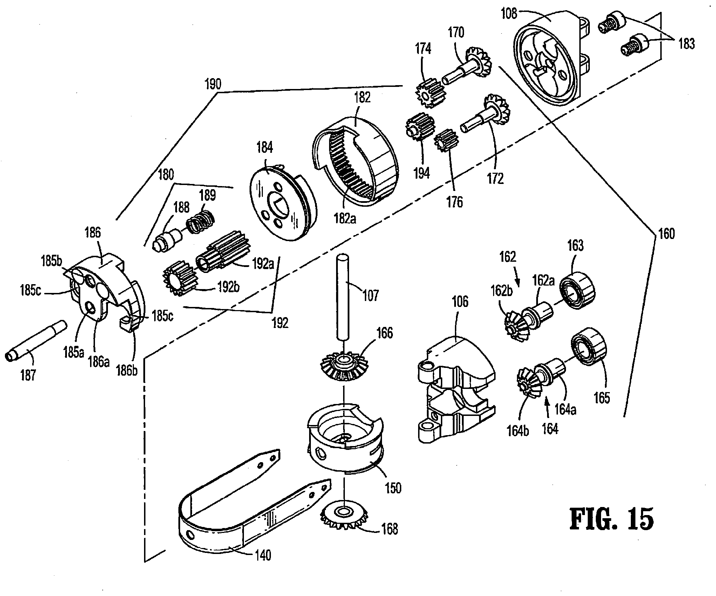

The proximal body portion 102 of the adapter assembly 100 includes the drive coupling assembly 110 (FIG. 5) and an articulation assembly 130 (FIG. 12) operably connected to the drive coupling assembly 110 and maintained within an outer sleeve 103 of the proximal body portion 102. A drive transfer assembly 160 (FIG. 15) extends between the proximal and distal body portions 102, 104 of the adapter assembly 100 and operably connects the drive coupling assembly 110 to a drive assembly 190 (FIG. 15) disposed within the distal body portion 104 of the adapter assembly 100.

With reference to FIGS. 4-10, as shown, the drive coupling assembly 110 has a cylindrical profile and is configured to selectively secure the adapter assembly 100 to the handle assembly 10 (FIG. 1). The drive coupling assembly 110 includes a connector housing 112 and a connector extension 114 fixedly connected to the connector housing 112 by an annular ring 113. The annular ring 113 may include a groove 113a or other feature for securing the adapter assembly 100 to the handle assembly 10. The connector extension 114 may include a notch 114a or other feature formed in an outer surface thereof for facilitating alignment of the adapter assembly 100 with the connector portion 18a of the handle assembly 10.

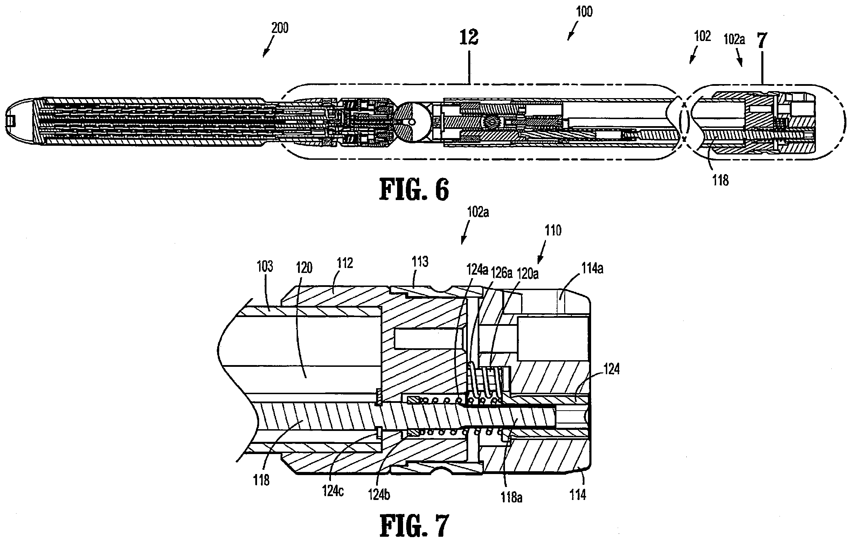

The connector housing 112 and the connector extension 114 operate to rotatably support a first drive shaft 116 (FIG. 5), a second drive shaft 118 (FIG. 7), and a third drive shaft 120 (FIG. 5). The connector housing 112 and the connector extension 114 of the drive coupling assembly 110 also rotatably support the first, second, and third connector sleeves 122 (FIG. 5), 124 (FIG. 7), and 126 (FIG. 5), respectively. The first, second, and third connector sleeves 122, 124, and 126 are configured to mate with the respective first, second, and third drive connectors (not shown) of the handle assembly 10 (FIG. 1) and are further configured to mate with a proximal end 116a, 118a, 120a of the respective first, second, and third drive shafts 116 (FIG. 5), 118 (FIG. 7), and 120 (FIG. 5) of the adapter assembly 100.

The drive coupling assembly 110 also includes first, second and third biasing members 122a (FIG. 5), 124a (FIG. 7), and 126a (FIG. 5) disposed distally of the respective first, second, and third connector sleeves 122, 124, and 126. The first, second, and third biasing members 122a, 124a, 126a are disposed about the respective first, second, and third drive shafts 116, 118, and 120 to facilitate engagement of the respective first, second, and third connector sleeves 122, 124, and 126 with respective first, second, and third rotatable drive connectors (not shown) of the handle assembly 10 (FIG. 1) when the adapter assembly 100 is connected to the handle assembly 10. In particular, the first, second, and third biasing members 122a, 124a, and 126a function to bias the respective first, second, and third connector sleeves 122, 124, and 126 in a proximal direction. First, second, and third washers 122b, 124b, and 126b are received about the respective proximal ends 116a, 118a, and 120a of the respective first, second, and third drive shafts 116, 118, and 120 to maintain the respective first, second, and third biasing members 122a, 124a, and 126a relative to the respective first, second, and third drive shafts 116, 118, and 120. A spring clip 124c (FIG. 7) is received about the second drive shaft 118 adjacent the proximal end 118a of the second drive shaft 118 to secure the second drive shaft 118 relative to the connector housing 112 of the drive coupling assembly 110.

For a detailed description of an exemplary drive coupling assembly, reference may be made to the '329 application.

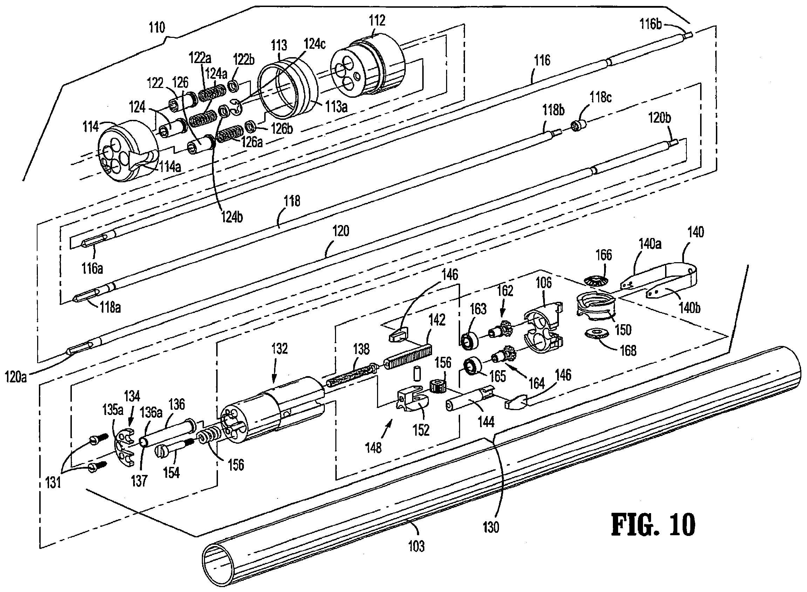

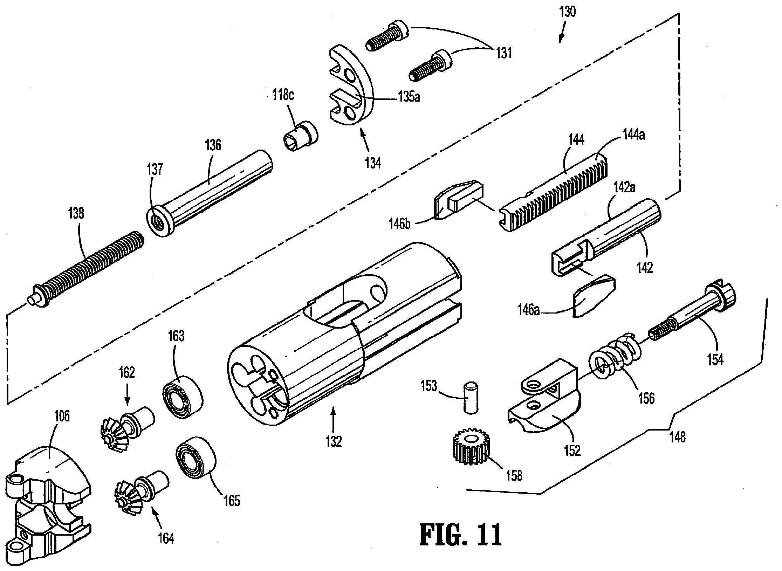

With reference now to FIGS. 10-16, the articulation assembly 130 effects articulation of the distal body portion 104 (FIG. 3) of the adapter assembly 100 relative to the proximal body portion 102 (FIG. 3) of the adapter assembly 100. With particular reference to FIGS. 10 and 11, the articulation assembly 130 includes an articulation housing 132 and a support plate 134 secured to a proximal end of the articulation housing 132 by a pair of screws 131. A connector tube 136 extends through an opening 135a in the support plate 134 and includes a threaded inner surface 137 for receiving a threaded connector screw 138. A proximal end 136a of the connector tube 136 operably engages an adapter member 118c that is secured to the distal end 118b (FIG. 10) of the second drive shaft 118.

The articulation assembly 130 further includes a flexible band 140 slidably disposed relative to the articulation housing 132. A first end 140a of the flexible band 140 is secured to the connector screw 138 by a first connector 142 and a second end 140b of the flexible band 140 is secured to a second connector 144. More particularly, the first and second connectors 142, 144 are each secured to the flexible band 140 by first and second adaptors 146a, 146b which are fixedly secured to the first and second ends 140a, 140b, respectively, of the flexible band 140 and operably secured to the respective first and second connectors 142, 144. The first and second adaptors 146a, 146b may be secured to the flexible band 140 by adhesive, welding, mechanical fasteners, or in any other suitable manner. Alternatively, the first and second connectors 142, 144 are directly secured to the flexible band 140 without the use of the first and second adaptors 146a, 146b. Each of the first and second connectors 142, 144 may be in the form of a gear rack each including a toothed surface 142a, 144a for operable engagement with a tensioning mechanism 148.

Turning briefly to FIG. 15, the flexible band 140 is received about a pivot member 150. The pivot member 150 is secured between the proximal connector housing 106 of the proximal body portion 102 and the distal connector housing 108 of the distal body portion 104 by the pivot pin 107. The pivot member 150 is pivotal relative to the proximal connector housing 106 and is fixedly secured to the distal connector housing 108. Rotation of the pivot member 150 about the pivot pin 107 through operation of the articulation assembly 130, i.e., longitudinal movement of the flexible band 140 relative to the pivot member 150, causes articulation of the distal body portion 104 of the adapter assembly 100 relative to the proximal body portion 102 of the adapter assembly 100.

During use, the articulation assembly 130 is actuated through rotation of the second drive shaft 118. In particular, rotation of the second drive shaft 118 causes rotation of the connector tube 136. Rotation of the connector tube 136 causes longitudinally movement of the connector screw 138. When the connector tube 136 is rotated in a first direction, the connector screw 138 is moved longitudinally in a proximal direction and when the connector tube 136 is rotated in a second direction (opposite the first direction), the connector screw 138 is moved in a distal direction. As noted above, the connector screw 138 is connected to the first connector 142 and the first connector 142 is connected to the flexible band 140. Thus, longitudinal movement of the first connector 142 causes corresponding movement of the flexible band 140. Since the flexible band 140 is received or fixedly connected about the pivot member 150, longitudinal movement of the flexible band 140 causes corresponding pivoting of the pivot member 150.

With continued reference to FIGS. 10-14, the flexible band 140 is maintained in tension about the pivot member 146 by the tensioning mechanism 148 received between the first and second connectors 142, 144 of the articulation assembly 130. The tensioning mechanism 148 includes a tensioning housing 152, a tensioning screw 154, a biasing member 156, and a tensioning gear 158 rotatably received within the tensioning housing 152 by a pivot pin 153. The tensioning housing 152 is adjustably secured to the tensioning screw 154 such that the tensioning housing 152 is slidably disposed within the articulation housing 132 between the first and second connectors 142, 144. The biasing member 156 is received about the tensioning screw 154 and engages the articulation housing 132. The biasing member 156 applies a force on the tensioning screw 154 which pulls the tensioning housing 152 in a proximal direction. The force applied by the biasing member 156 on the tensioning screw 154 can be adjusted by rotating the tensioning screw 154 and acts on the first and second connectors 142, 144 through the tensioning gear 158 to keep the first and second connectors 142, 144 and the flexible band 140 in tension, thereby ensuring that the first and second connectors 142, 144 move uniformly during operation of the articulation assembly 130. In this manner, the tensioning mechanism 148 ensures that the first connector 142 is advanced or retracted at the same rate as the second connector 144 is retracted or advanced. The tensioning mechanism 148 may also operate to maintain frictional engagement between the flexible band 140 and the pivot member 150. Alternatively, the flexible band 140 is secured to the pivot member 150 using an adhesive, mechanical fastener or other in any other suitable manner.

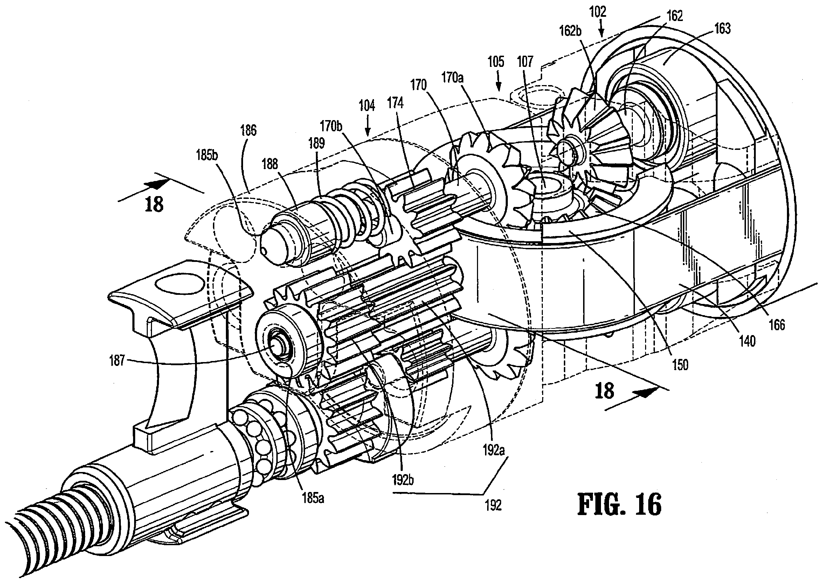

With particular reference again to FIG. 15 and additional reference to FIGS. 16-18, the drive transfer assembly 160 operably connects the drive coupling assembly 110 (FIG. 10) with the drive assembly 190. The drive transfer assembly 160 includes first and second proximal bevel gears 162, 164, first and second horizontal bevel gears 166, 168, and first and second distal bevel gears 170, 172. The first proximal bevel gear 162 is rotatably supported within the proximal connector housing 106 by a first bearing assembly 163. A first end 162a of the first proximal bevel gear 162 operably engages the distal end 116b (FIG. 10) of the first drive shaft 116 of the drive coupling assembly 110 and a second end 162b of the first proximal bevel gear 162 engages the first horizontal bevel gear 166. The second proximal bevel gear 164 is rotatably supported within the proximal connector housing 106 by a second bearing assembly 165. A first end 164a of the second proximal bevel gear 164 operably engages the distal end 120b (FIG. 10) of the third drive shaft 120 of the drive coupling assembly 110 and a second end 164b of the second proximal bevel gear 164 engages the second horizontal bevel gear 168.

The first and second horizontal bevel gears 166, 168 are rotatably supported within the pivot member 150 of the articulation assembly 130 (FIG. 10) about the pivot pin 107. The first horizontal bevel gear 166 operably engages a first end 170a of the first distal bevel gear 170 and the second horizontal bevel gear 168 operably engages a first end 172a of the second distal bevel gear 170. The first and second distal bevel gears 170, 172 are rotatably supported within the distal connector housing 108. A second end 170b of the first distal bevel gear 170 includes or supports a first spur gear 174 and a second end 172b of the second distal bevel gear 172 includes or supports a second spur gear 176. In this manner, power transmission between the proximal and distal body portions 102, 104 occurs within the articulation joint 105.

The drive transfer assembly 160 operates to transfer the rotational motion from the first and third drive shafts 116, 120 (FIG. 10) of the drive coupling assembly 110 in the proximal body portion 102 of the adapter assembly 100 to rotational motion of the respective first and second spur gears 174, 176 within the distal body portion 104 of the adapter assembly 100. As will be described in further detail below, rotation of the first spur gear 174 causes actuation of the loading unit 200 (FIG. 1) and rotation of the second spur gear 176 causes rotation of the loading unit 200 (FIG. 1) about the longitudinal axis "x" of the adapter assembly 100.

With particular reference to FIG. 16, the first drive shaft 116 (FIG. 10) rotates the first proximal bevel gear 162 in a first direction about a first longitudinal axis (not shown) extending parallel to the longitudinal axis "x" of the adapter assembly 100. Engagement between the first proximal bevel gear 162 and the first horizontal bevel gear 166 causes the first horizontal bevel gear 166 to rotate about a longitudinal axis (not shown) of the pivot pin 107, i.e., perpendicular to the longitudinal axis "x" of the adapter assembly 100. Engagement between the first horizontal bevel gear 166 and the first distal bevel gear 170 causes rotation of the first spur gear 174 in a second direction about the first longitudinal axis (not shown) of the adapter assembly 100 when the proximal and distal body portions 102, 104 (FIG. 3) of the adapter assembly 100 are axially aligned. The drive transfer assembly 160 permits the transfer of rotational motion from the first drive shaft 116 to the first spur gear 174 throughout articulation of the distal body portion 104 of the adapter assembly 100 relative to the proximal body portion 102 of the adapter assembly 100.

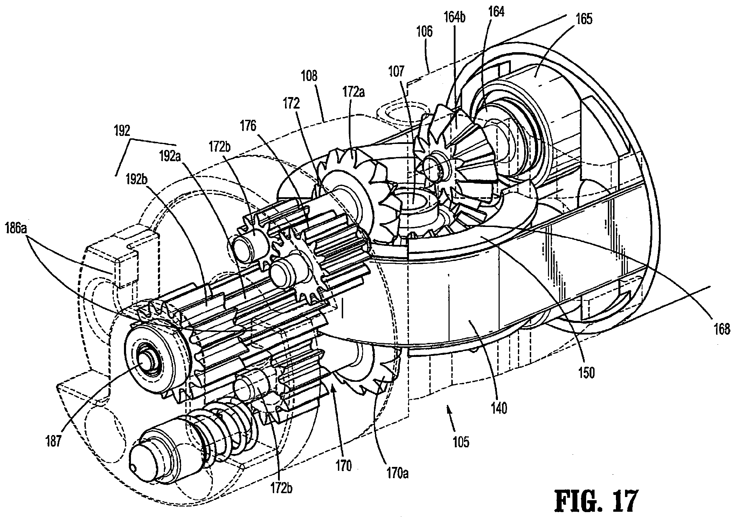

With particular reference to FIG. 17, the third drive shaft 120 (FIG. 10) rotates the second proximal bevel gear 164 in a first direction about a second longitudinal axis (not shown) extending parallel to the longitudinal axis "x" of the adapter assembly 100. Engagement between the second proximal bevel gear 164 and the second horizontal bevel gear 168 causes the second horizontal bevel gear 168 to rotate about the longitudinal axis (not shown) of the pivot pin 107, i.e., perpendicular to the longitudinal axis "x" of the adapter assembly 100. Engagement between the second horizontal bevel gear 168 and the second distal bevel gear 172 causes rotation of the second spur gear 176 in a second direction about the second longitudinal axis (not shown) of the adapter assembly 100 when the proximal and distal body portions 102, 104 of the adapter assembly 100 are axially aligned. The drive transfer assembly 160 permits the transfer of rotational motion from the third drive shaft 120 to the second distal spur gear 176 throughout articulation of the distal body portion 104 of the adapter assembly 100 relative to the proximal body portion 102 of the adapter assembly 100.

With continued reference to FIGS. 15-17, the distal body portion 104 of the adapter assembly 100 includes a cylindrical housing 182 rotatably supported relative to the distal connector housing 108, a support plate 184 securely supported on or connected to the distal connector housing 108, and a latch housing 186 securely supported to the cylindrical housing 182. The cylindrical housing 182 includes a toothed inner surface 182a for effecting rotation of the cylindrical housing 182 and the latch housing 186. Although shown secured to the distal connector housing 108 by a pair of screws 183, the support plate 184 may be secured to the distal connector housing 108 in any suitable manner.

The latch housing 186 of the distal body portion 104 of the adapter assembly 100 is configured for selective connection with the loading unit 200. More particularly, the latch housing 186 includes a tongue 186a and a pair of inwardly extending lips 186b. As will be described in further detail below, the tongue 186a and the lips 186b cooperate to engage the loading unit 200. The latch housing 186 defines a first longitudinal opening 185a for receiving a pivot pin 187 and a second longitudinal opening 185b for operably receiving a latch member 188 and a biasing member 189. Biasing member 189 may include a compression spring, as shown in FIG. 15, or any other means capable of biasing the latch member 188 in a distal direction. As will be described in further detail below, the latch housing 186 further includes a pair of bores 185c for receiving a pair of locking members 252 that selectively extend from the loading unit 200 to prevent separation of the loading unit 200 from the adapter assembly 100 when the cartridge assembly 300 is received within the loading unit 200.

The distal body portion 104 of the adapter assembly 100 operably supports the drive assembly 190 of the adapter assembly 100. The drive assembly 190 includes a drive gear assembly 192 rotatably received about the pivot pin 187. The drive gear assembly 192 includes a primary drive gear 192a and a secondary drive gear 192b fixedly secured relative to the primary drive gear 192a such that the secondary drive gear 192b rotates as the primary drive gear 192a is rotated. The drive gear assembly 190 further includes a rotation drive gear 194 for causing rotation of the cylindrical housing 182 and the latch housing 186 relative to the distal connector housing 108 of the distal body portion 104. When the loading unit 200 is secured to the latch housing 182, rotation of the drive gear 194 causes rotation of the loading unit 200.

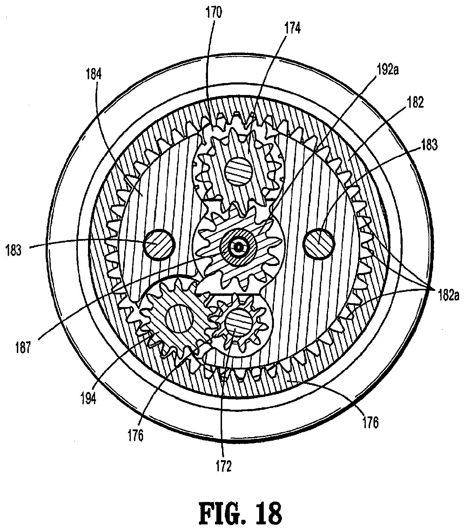

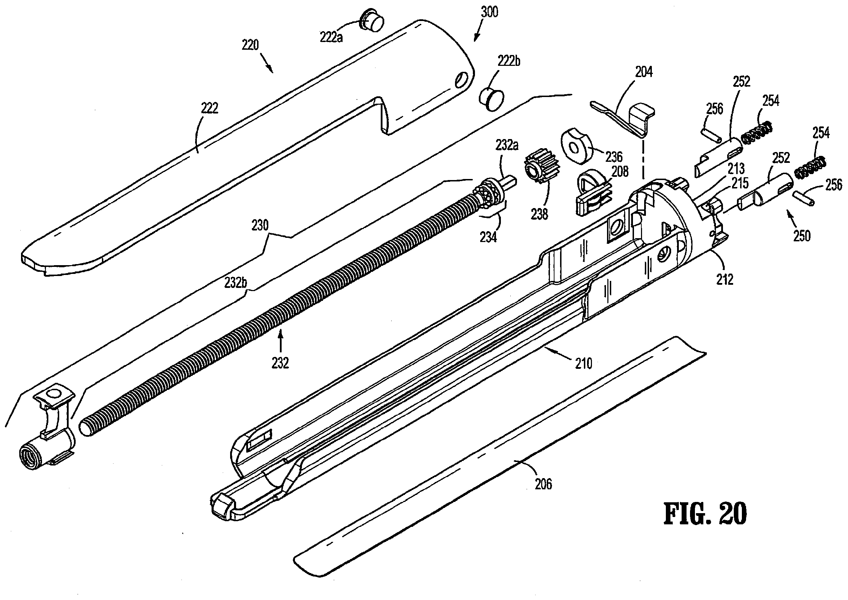

As shown in FIG. 18, the primary drive gear 192a of the drive gear assembly 192 engages the first spur gear 174 mounted on the second end 170b of the first distal bevel gear 170 of the drive transfer assembly 160 (FIG. 15). Rotation of the first spur gear 174, as described above, causes rotation of the primary and secondary drive gears 192a, 192b. The latch housing 186 is configured such that a portion of the secondary drive gear 192b is exposed. As will be described in further detail below, when the loading unit 200 is secured to the latch housing 186 of the distal body portion 104 of the adapter assembly 100, the secondary drive gear 192b engages a drive gear 238 (FIG. 20) of an actuation assembly 230 (FIG. 20) of the loading unit 200 to enable actuation of the loading unit 200.

With continued reference to FIG. 18, the rotation drive gear 194 engages the second spur gear 176 mounted on the second end 172b of the second distal bevel gear 170 of the drive transfer assembly 160 (FIG. 15) and the toothed surface 182a of cylindrical housing 182. Rotation of the second spur gear 176, as described above, causes rotation of the rotation drive gear 194 which causes the rotation of the cylindrical housing 182 and the latch housing 186 and further causes rotation of the loading unit 200 secured to the latch housing 186.

As shown in FIG. 18, the primary drive gear 192a of the drive gear assembly 192 and the first spur gear 174 for driving the primary drive gear 192, as well as the rotation drive gear 194 and the second spur gear 172 for driving the rotation drive gear 194 are disposed within the same cross-sectional plane of the distal body portion 104. This compact design of the drive transfer assembly 160 and the drive assembly 190 and the location of the power transmission within the articulation joint 105 allows for a shortened length of the distal body portion 104. The shortened length of the distal body portion 104 allows for increased manipulability of an end effector, e.g., the loading unit 200, during a surgical procedure.

As shown, the length of the distal body portion 104 is substantially equal to the diameter of the distal body portion 104. Alternatively, the length of the distal body portion 104 may be less than the diameter of the distal body portion 104. In this manner, the secondary drive gear 192b of the drive gear assembly 192 is disposed within one diameter length of the articulation joint 105. For example, if the diameter of the distal body portion 104 is fifteen millimeters (15 mm), the distance between the pivot pin 107 of the articulation joint 105 and the primary drive gear 192a is less than fifteen millimeters (15 mm). In embodiments, the distance between the pivot pin 107 and the secondary drive gear 192b is between five millimeters (5 mm) and thirty millimeters (30 mm), and more specifically, between ten millimeters (10 mm) and twenty millimeters (20 mm).

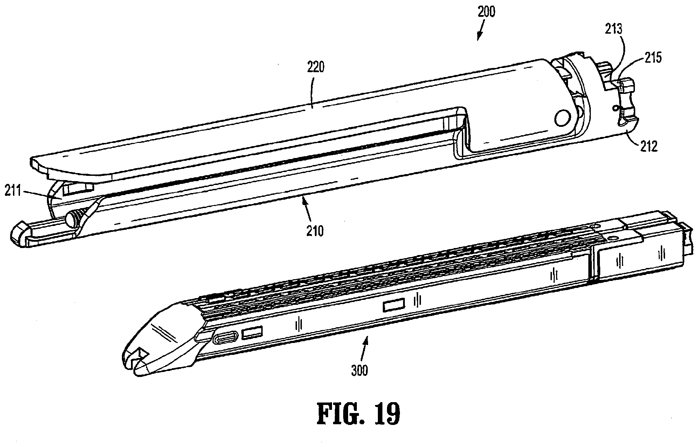

The loading unit 200 and the cartridge assembly 300 will be described with reference to FIGS. 19-31. The loading unit 200 includes a carrier 210, an anvil assembly 220, an actuation assembly 230, and a locking mechanism 250. The carrier 210 and an anvil member 222 of the anvil assembly 220 are pinned together by a pair of pins 222a, 222b and are movable between open (FIG. 4) and closed (FIG. 19) positions. The anvil member 222 is biased to the open position by a leaf spring 204. The carrier 210 defines a longitudinal recess 211 for operably supporting the actuation assembly 230 and selectively receiving the cartridge assembly 300. Optionally, an insert or pad 206 is received with the longitudinal recess 211 of the carrier 210 between the carrier 210 and the cartridge assembly 300 to reduce the friction between a drive beam 240 of the actuation assembly 230 and the carrier 210 during operation of the loading unit 200. An electrical contact member 208 is supported in the carrier 210 and is in electrical communication with the handle assembly 10 (FIG. 1) through the adapter assembly 100 (FIG. 1) when the loading unit 200 is secured to the adapter assembly 100.

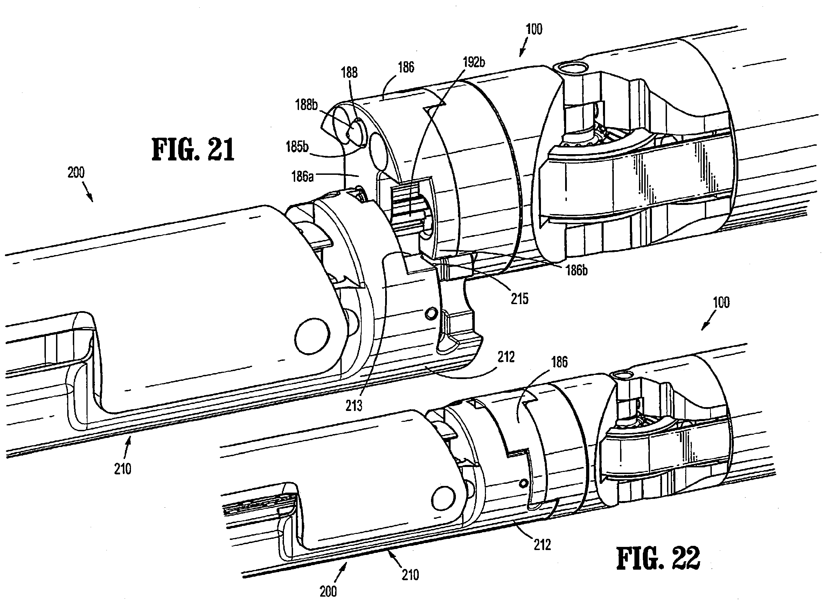

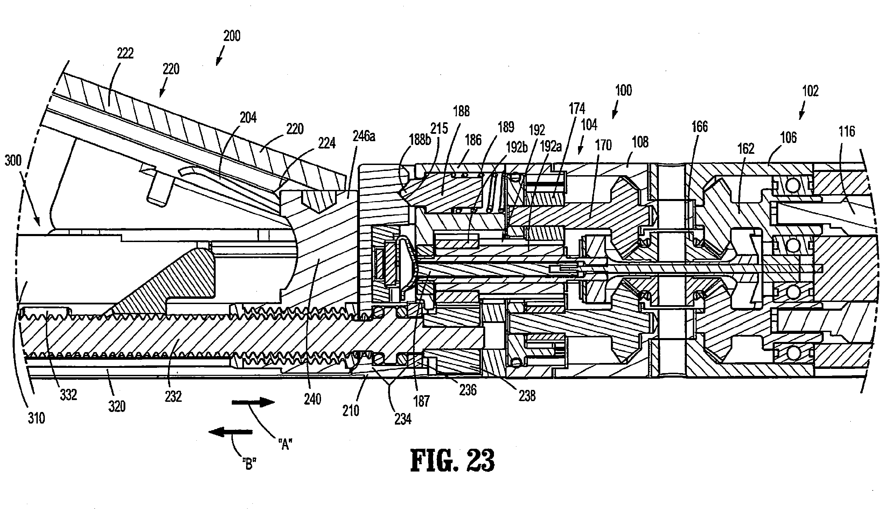

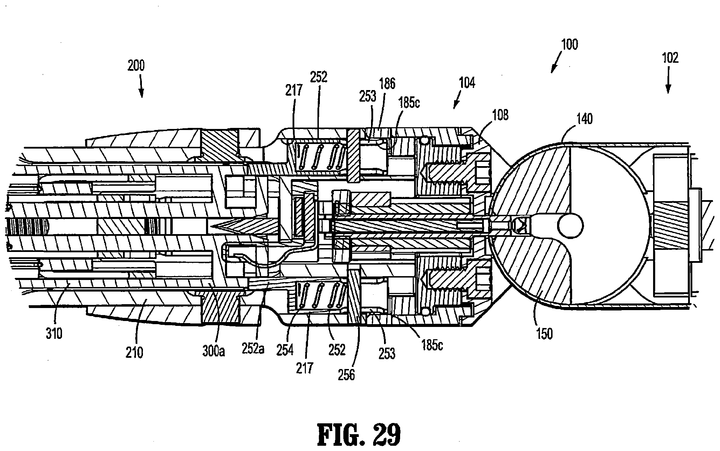

With particular reference now to FIGS. 21-23, a proximal end of the carrier 210 is configured to selectively secure loading unit 200 to adapter assembly 100. Specifically, the carrier 210 includes an extension 212 that engages the latch housing 186 of adapter assembly 100. The extension 212 defines a cutout 213 for receiving the tongue 186a of the latch housing 186 of the adapter assembly 100. The extension 212 further defines a pair of notches 213 for receiving the lips 186b of the latch housing 186 of the adapter assembly 100. The extension 212 still further defines a recess 215 (FIG. 23) for receiving the distal end 188b of the latch member 188 of the latch mechanism 180 of the adapter assembly 100 when the loading unit 200 is engaged with the adapter assembly 100 for frictionally securing the loading unit 200 to the adapter assembly 100. The extension 212 also defines a pair of longitudinal bores 217 (FIG. 29) for operably receiving locking members 252 and biasing members 254 of a locking mechanism 250, as will be described below.

With particular reference to FIGS. 21 and 22, the loading unit 200 is secured to the adapter assembly 100 by aligning the tongue 186a of the latch housing 186 of the adapter assembly 100 with the cutout 213 of the carrier 210 of the loading unit 200 and the lips 186b of the latch housing 186 of the adapter assembly 100 with the notches 215 of the carrier 210 of the loading unit 200 and moving the loading unit 200 relative to the adapter assembly 100. The tongue 186a and lips 186b of the latch housing 186 are received within the respective cutout 213 and notches 215 until the distal end 188b of the latch member 188 of the adapter assembly 100 aligns with and is subsequently received within the recess 215 in the carrier 210 of the loading unit 200. The distal end 188b of the latch member 188 may be conical, as shown in FIG. 21, to facilitate retraction of the latch member 188 within the second longitudinal opening 185b of the latch housing 186 against the bias of biasing member 189 (FIG. 23) as the tongue 186a and lips 186b of the latch housing 186 are received within the respective cutout 213 and notches 215 in the carrier 210 of the loading unit 200.

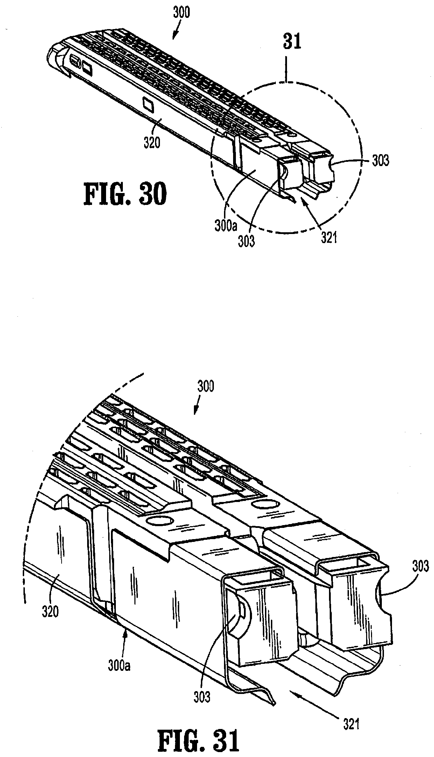

The receipt of the distal end 188b of the latch member 188 in the recess 215 of the carrier 210 of the loading unit 200 frictionally secures the loading unit 200 with the adapter assembly 100. The receipt of the distal end 188b of the latch member 188 in the recess 215 may produce a tactile and/or audible feedback to the user indicating that the loading unit 200 is securely attached to the adapter assembly 100.Prior to the cartridge assembly 300 (FIG. 30) being received within the longitudinal recess 211 of the carrier 210 of the loading unit 200 and/or once the cartridge assembly 300 has been removed from the carrier 210, the loading unit 200 may be separated from the adapter assembly 100 by overcoming the friction force provided by the latch member 188 of the latch mechanism 180.

With continued reference to FIG. 20-23, the actuation assembly 230 of the loading unit 200 includes a lead screw 232 rotatably supported within the carrier 210 by a bearing assembly 234 and a bearing member 236. A drive gear 238 is supported on a proximal end 232a of the drive screw 232 between the bearing assembly 234 and the bearing member 236. The drive gear 238 engages the secondary drive gear 192b (FIG. 21) of the adapter assembly 100 when the loading unit 200 is secured to the adapter assembly 100 and functions to transfer the rotational motion from the secondary drive gear 192b to the drive screw 232 of the loading unit 200. A distal end 232b of the drive screw 232 is threaded and supports a drive beam 240 thereon.

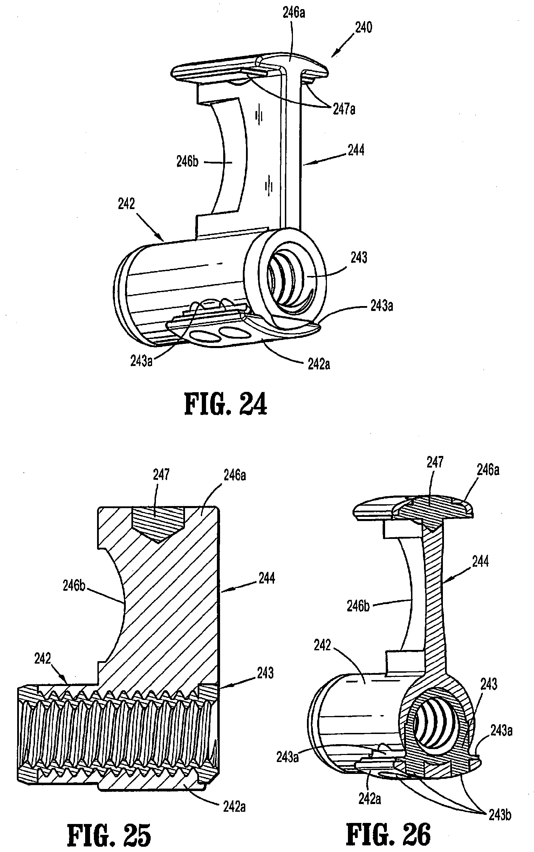

Turning briefly to FIGS. 24-26, the drive beam 240 of the loading unit 200 includes a retention portion 242 and a vertical support portion 244. The retention portion 242 of the drive beam 240 is configured for threaded engagement with the threaded distal end 232b of the drive screw 232 (FIG. 20). A flange 242a formed on the retention portion 242 of the drive beam 240 maintains the drive beam 240 in a vertical orientation relative to the carrier 210 (FIG. 20) and the staple cartridge 300 (FIG. 19). The vertical support portion 244 of the drive beam 240 includes a cam member 246a and a knife member 246b. The cam member 246a is formed on a free end of the vertical support portion 244 of the drive beam 240 and is configured for selective engagement with a ledge 224 (FIG. 23) of the anvil member 222 of the anvil assembly 220. As will be described in further detail below, engagement of the cam member 246a of the drive beam 240 with the ledge 224 of the anvil member 222 causes anvil member 222 to move from the open position (FIG. 4) to the closed position (FIG. 19) and maintains the anvil member 222 in the closed position. The knife member 246b of the vertical support portion 244 of the drive beam 240 is configured to cut the tissue (not shown) clamped between the cartridge assembly 300 (FIG. 19) and the anvil member 222 (FIG. 19) during actuation of the loading unit 200.

In embodiments, and as shown, the drive beam 240 is formed of metal, e.g., stainless steel, or other suitable material. The retention portion 242 and the cam member 246a of the drive beam 240 each include a molded insert 243, 247, respectively, formed of plastic, e.g., peek, or other suitable material. The molded inserts 243, 247 are co-molded with the respective retention portion 242 and the cam member 246a of the drive beam 240. The molded insert 243 includes wings 243a formed on upper surfaces of the flange 242a of the retention portion 242 on either side of the retention portion 242. The wings 243a operate to reduce the friction between the drive member 240 and the carrier 210 of the loading unit 200, thereby reducing the input torque necessary to advance the drive beam 240 through the carrier 210. Similarly, the molded insert 247 includes wings 247a on an undersurface of the cam member 246a on either side of the vertical support portion 244 of the drive beam 240. The wings 247a reduce friction between the drive beam 240 and the anvil member 222, thereby reducing the input torque necessary to advance the drive beam 240 through the carrier 210 of the loading unit 200. The stainless steel portions of the drive member 240 provide sufficient strength to support the loads required to clamp and fire a plurality of staples (not shown).

As shown in FIG. 25, in addition to the threads provided in the molded insert 243, the retention portion 242 of the drive beam 240 is also provided with threads. The threads of the retention portion 242 correspond to the threads of the molded insert 243 and operate to reinforce the threads of the molded insert. In this manner, the threads of the retention portion 242 strengthen the engagement between the drive beam 240 and the lead screw 232, thereby reducing the likelihood of failure during operation of the loading unit 200.

Turning to FIG. 26, the molded insert 243 of the drive beam 240 is provided with anti-rotation features 243b. The anti-rotation features 243a extends through flange 242a of retention portion 242 to fix molded insert 243 within retention portion 242 and prevent rotation of the molded insert 243 during advancement and retraction of the drive beam 240.With particular reference now to FIG. 23, the loading unit 200 may be provided to the surgeon with the anvil member 222 of the anvil assembly 200 in the open position (FIG. 4) or in the closed position (FIG. 19). When provided to the surgeon in the closed position, following attachment of the loading unit 200 to the adapter assembly 100, the loading unit 200 is moved the open position to permit loading of the cartridge assembly 300 within the longitudinal recess 211 of the carrier 210. As noted above, the anvil member 222 of the loading unit is maintained in the closed position through engagement of the cam member 246a of the drive member 240 with the ledge 224 of the anvil member 222. Accordingly, to move the loading unit 200 to the open position the drive beam 240 is moved proximally, i.e., retracted, as indicated by arrow "A" in FIG. 23, to disengage the cam member 246a of the drive beam 240 from the ledge 224 of the anvil member 222.

As described above, the drive beam 240 is translated longitudinally relative to the drive screw 232 by rotating the first drive shaft 116, which rotates the first proximal bevel gear 162, which rotates the first horizontal bevel gear 166, which rotates the first distal bevel gear 170, which rotates of the first spur gear 174, which rotates the primary drive gear 192a thereby rotating the secondary drive gear 192b, which rotates the drive gear 238 of the loading unit 200, which rotates the drive screw 232 to move the drive beam 240. Rotation of the first drive shaft 116 in a first direction causes the drive beam 240 to move in the proximal direction and rotation of the first drive shaft 116 in the second direction causes the drive beam 240 to move in a distal direction, i.e., advance, as indicated by arrow "B" in FIG. 23.

Proximal movement of the drive beam 240 causes the cam member 246a of the drive beam 240 to disengage from the ledge 224 of the anvil member 222. Once the cam member 246a of the drive beam disengages the anvil member 222, the leaf spring 204 biases the anvil member 222 to the open position. Once open, the cartridge assembly 300 is received within the longitudinal passage 211 of the carrier 210 of the loading unit 200.

The loading unit 200 may then be received through an access port (not shown) and positioned about tissue to be stapled (not shown) in a traditional manner. At any point during the stapling procedure, as described in detail above, the loading unit 200 may be articulated relative to the proximal body portion 102 of the adapter assembly 100 and/or the loading unit 200 may be rotated about the longitudinal axis "x" of the adapter assembly 100 for positioning the loading unit 200.

With reference still to FIG. 23, once tissue to be stapled (not shown) is received between the anvil member 222 of the anvil assembly 220 and the cartridge member 310 of the cartridge assembly 300, rotation of the first drive shaft 116 of the adapter assembly 100 in a second direction causes the drive beam 240 of the loading unit 200 to move distally. As the drive beam 240 moves distally, the cam member 246a of the drive beam 240 engages the ledge 224 of the anvil member 220 to cause the closing of anvil member 220. Continued distal movement of the drive beam 240 effects movement of an actuation sled 330 of the cartridge assembly 300 into pusher members 332 to cause the stapling of tissue (not shown). As the drive member 240 is moved distally, the knife member 246b engages and cuts the stapled tissue (not shown). Once the tissue (not shown) is stapled and cut, the drive beam 240 is retracted through rotation of the first drive shaft 116 in the first direction, as described above.

The loading unit 200 may then be removed from the patient and the spent cartridge assembly 300 may be separated or unloaded from the carrier 210. Once the spent cartridge assembly 300 is separated from the carrier 210, the loading unit 200 may be separated from the adapter assembly 100 and discarded. Alternatively, a new cartridge assembly 300 may be attached to or loaded into the loading unit 200 to permit reuse of the loading unit 200.

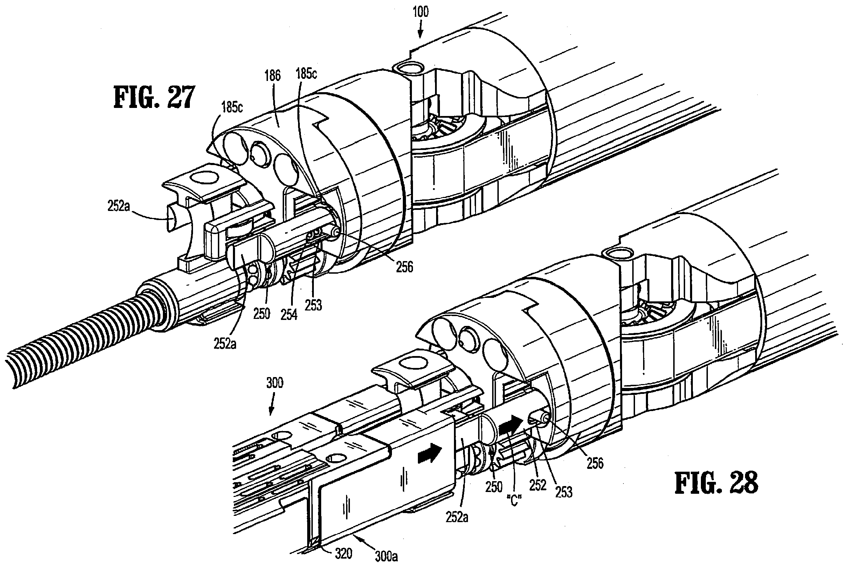

With reference now to FIGS. 27-31, as noted above, the loading unit 200 includes a locking mechanism 250 for preventing separation of the loading unit 200 from the adapter assembly 100 when the cartridge assembly 300 is secured within the loading unit 200. The locking mechanism 250 includes a pair of locking member 252 and a pair of biasing members, i.e., springs 254, received within the locking members 252. Pins 256 extend through slots 253 in the locking members 252 to retain the biasing members 254 about the locking members 252 and to support the locking members 252 within the longitudinal bores 217 of the extension 212 of the carrier 210. The locking members 252 include distal projections 252a that extend into the longitudinal recess 211 of the carrier 210 and engage a proximal end 300a of the cartridge assembly 300 when the cartridge assembly 300 is operably secured to the carrier 210. As shown in FIG. 31, a proximal end of a cartridge body 310 of the cartridge assembly 300 includes a pair of notches 313 for receiving the distal projections 252a of the locking members 252.

With particular reference now to FIG. 27, when the locking mechanism 250 is in a first position, i.e., prior to the cartridge assembly 300 being received within the longitudinal recess 211 of the carrier 210 of the loading unit 200, the locking members 252 are in a distal-most position. When in the distal-most position, the loading unit 200 is separable from the adapter assembly 100, as described above.

Turning to FIGS. 28 and 29, when the cartridge assembly 300 is received within the longitudinal recess 211 of the carrier 210, the proximal end 300a of the cartridge assembly 300 engages the distal projections 252a of the locking members 252 causing the locking members 252 to move in a proximal direction, as indicated by arrow "C" in FIG. 28, and into the bores 185c of the latch housing 186 of the adapter assembly 100. Receipt of the locking member 252 within the bores 185c of the latch housing 186 secure the loading unit 200 to the adapter assembly 100 and prevent the loading unit 200 from accidently detaching from the adapter assembly 100 during operation of the loading unit 200.

Turning briefly to FIGS. 30 and 31, the cartridge assembly 300 includes the cartridge body 310 and a cartridge housing 320. The cartridge housing 320 securely receives the cartridge body 310 and defines a longitudinal slot 321 for receiving the drive screw 232 of the actuation assembly 230 of the loading unit 200 therethrough. As noted above, the cartridge assembly 300 further includes the actuation sled 330 and the pusher members 332. For a detailed description of an exemplary cartridge assembly, please refer to commonly owned U.S. Patent Application Serial No. 14/257,063 (Publication number US 2015-0297199 A1) filed April 21, 2014, entitled "Adapter Assembly with Gimbal for Interconnecting Electromechanical Surgical Devices and Surgical Loading Units, and Surgical Systems Thereof,".

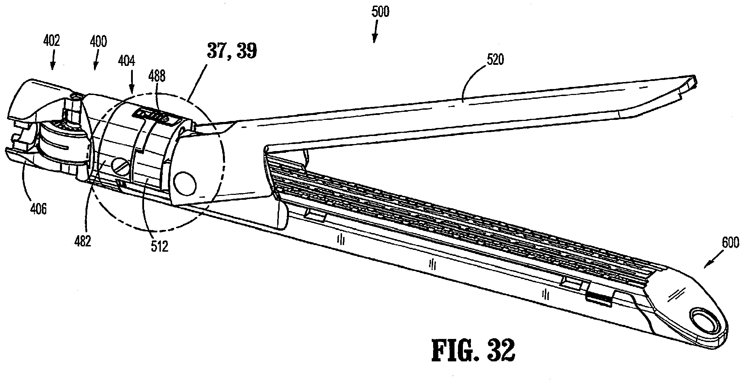

With reference now to FIGS. 32-40, an adapter assembly in accordance with another embodiment of the present disclosure, shown generally as adapter assembly 400, and a loading unit according to another embodiment of the present disclosure, shown generally as loading unit 500, are configured for selective connection to the handle assembly 10 (FIG. 1). The adapter assembly 400 is configured for selective connection with the handle assembly 10, and, the loading unit 500 is configured for selective connection with the adapter assembly 400. As will be shown and described in detail below, the loading unit 500 is a single use loading unit configured to fixedly receive a cartridge assembly 600; however, it is envisioned that the loading unit 500 may be modified to receive a replaceable cartridge assembly (not shown).

The adapter assembly 400, the loading unit 500 and the cartridge assembly 600 operate to staple and cut tissue. The adapter assembly 400 and the loading unit 500 are substantially similar to the adapter assembly 100 and the loading unit 200 described hereinabove, and therefore, will only be described to the extent necessary to detail the differences therebetween.

With reference initially to FIG. 32, a proximal body portion 402 (only a proximal connector housing 406 shown) and a distal body portion 404 of the adapter assembly 400 are substantially the same as the proximal body portion 102 (FIG. 3) and the distal body portion 404 (FIG. 3), respectively, of the adapter assembly 100 (FIG. 3) described hereinabove. Accordingly, the proximal and distal body portions 402, 404 will only be described in detail to the extent necessary to distinguish the differences therebetween.

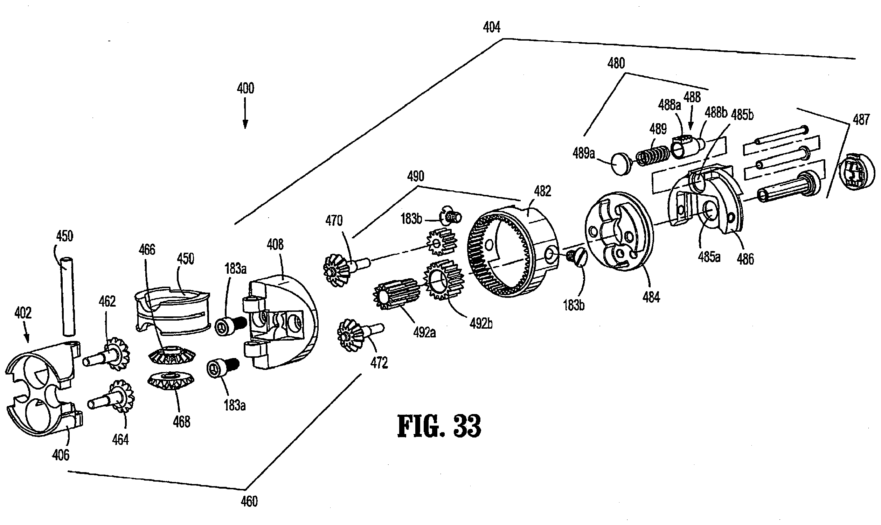

With reference now to FIGS. 32 and 33, a distal connector housing 408 of the distal body portion 404 is pivotally connected relative to the proximal connector housing 406 of the proximal body portion 402 by a pivot member 450. The adapter assembly 400 includes a drive transfer assembly 460 operably supported within proximal and distal connector housings 406, 408, and a drive assembly 490 operably supported within and extending through a cylindrical housing 482, support plate 484, and latch housing 486 of distal body portion 404. The support plate 484 is secured to the distal connector housing 408 by a pair of screws 483a and the latch housing 486 is secured to the cylindrical housing 482 by a pair of screws 483b. Alternatively, the support plate 484 and distal connector housing 408 and/or the latch housing and the cylindrical housing 482 may be secured to each other in any suitable manner, e.g., threading, friction fit, mechanical fasteners.

With particular reference to FIG. 33, briefly, the drive transfer assembly 460 includes first and second proximal bevel gears 462, 464, first and second horizontal bevel gears 466, 468, and first and second distal bevel gears 470, 472. The drive gear assembly 490 includes a primary drive gear 492a and a secondary drive gear 492b fixedly secured relative to the primary drive gear 492a, and a rotation drive gear (not shown). For a detailed description of the operation of the drive transfer assembly 460 and drive gear assembly 490, please refer to the drive transfer assembly 160 (FIG. 15) and the drive gear assembly 190 (FIG. 15) of adapter assembly 100 described hereinabove.

With continued reference to FIGS. 32 and 33, the latch housing 486 of the distal body portion 404 of the adapter assembly 400 is configured for selective connection with the loading unit 500 (FIG. 32). The latch housing 486 defines a first longitudinal opening 485a for receiving a bearing assembly 487 and a second longitudinal opening 485b for operably receiving a latch mechanism 480. The bearing assembly 487 rotatably supports the drive gear assembly 490 and the latch mechanism 480 selectively secures the loading unit 500 to the distal body portion 404 of the adapter assembly 400.

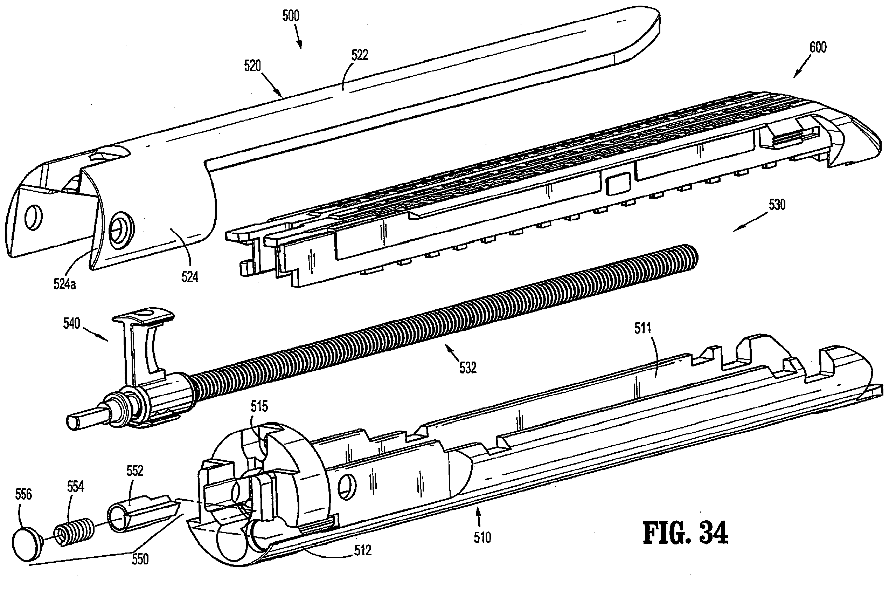

The latch mechanism 480 includes a latch member 488, a biasing member 489, and a cap member 489a for retaining the latch member 488 and the biasing member 489 within the second longitudinal opening 485b. The latch member 488 includes a proximal portion 488a configured for operable engagement by a user and a distal portion 488b configured to be selectively received within a recess 515 (FIG. 34) defined in an extension 512 of a carrier 510 of loading unit 500. The latch mechanism 480 operates to selective secure the loading unit 500 to the distal body portion 404 of the adapter assembly 400. Specifically, the biasing member 489 of the latch mechanism 480 biases the distal portion 488b of the latch member 488 into the recess 515 formed in the extension 512 of the carrier 510 of loading unit 500 when the loading unit 500 is engaged with the latch housing 486 of the adapter assembly 400 to prevent the loading unit 500 from disengaging from the latch housing 486 of the adapter assembly 400. Proximal movement of the latch member 488, against the bias of the biasing member 489, through engagement of the proximal portion 488a of the latch member 488 retracts the distal portion 488b of the latch member 488 from within the recess 515 in the extension 512 of the carrier 510 of the loading unit 500, thereby permitting the loading unit 500 to be separated from the adapter assembly 400 in the same manner loading unit 200 is separated from adapter assembly 100, described hereinabove.

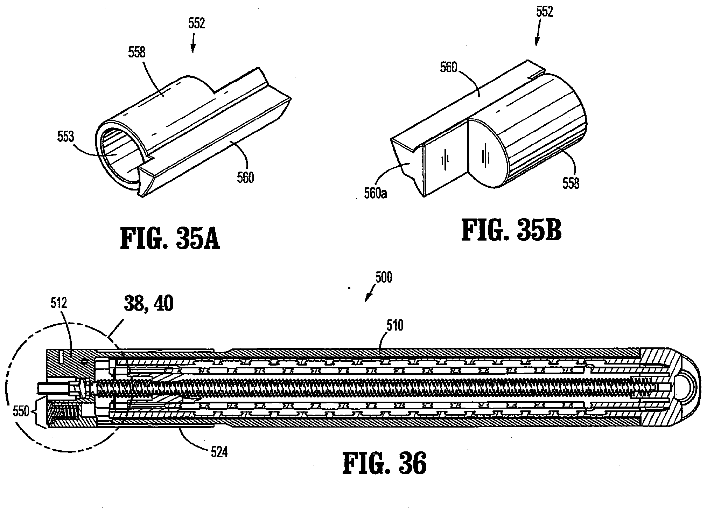

With reference now to FIG. 34, the loading unit 500 includes the carrier 510, an anvil assembly 520, an actuation assembly 530, and a plunger assembly 550. A distal end of the carrier 510 of the loading unit 500 defines a longitudinal recess 511 for receiving the staple cartridge 600. The cartridge assembly 600 may be permanently affixed within the longitudinal recess 511 of the carrier 510 such that the cartridge assembly 600 cannot be replaced, i.e., for single use, or the cartridge assembly 600 may be selectively secured with the longitudinal recess 511 of the carrier 510 to permit replacement of the cartridge assembly 600, i.e., for multiple uses. The anvil assembly 520 includes an anvil member 522 pivotally secured to the carrier 510 by a pair of pivot pins (not shown). The anvil member 522 includes a C-shaped frame portion 524. As will be described in further detail below, a proximal end 524a of C-shaped frame portion 524 is configured for operable engagement by a distal end 560a (FIG. 35B) of a longitudinal body portion 560 of a plunger member 552 of the plunger assembly 550.

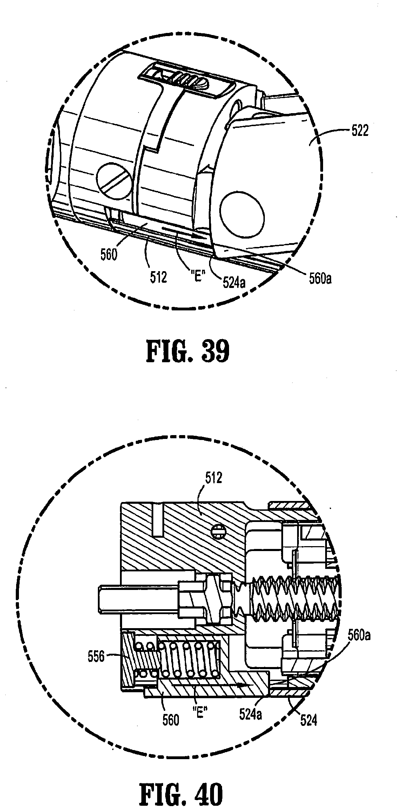

With continued reference to FIG. 34, the actuation assembly 530 includes the drive screw 532 and a drive beam 540. With additional reference to FIGS. 35A and 35B, the plunger assembly 550 is operably mounted within an extension 512 of the carrier 510 and includes a plunger member 552, a spring member 554, and an end cap 556. The plunger member 552 of the plunger assembly 550 is slidably disposed within the extension 512 of the carrier 510 of the loading unit 500 and is configured to bias the anvil member 522 of the anvil assembly 520 to an open position (FIG. 40). Specifically, the plunger member 552 includes a cylindrical body portion 558 received within a longitudinal bore 517 formed in the extension 512 of the carrier 510. A distal end 554b of the spring member 554 is received within a cylindrical recess 553 defined by the cylindrical body portion 558. The plunger member 552 and the spring member 554 are maintained within the longitudinal bore 517 of the extension 512 by the end cap 556. The end cap 556 may be secured to the extension 512 of the carrier 510 by friction fit, welding, adhesive, mechanical fasteners, or in any other suitable manner. Alternatively, the end cap 556 and the longitudinal bore 517 may be threaded or include a tab and slot arrangement for securing the end cap 556 to the extension 512 of the carrier 512.

The plunger member 552 of the plunger assembly 550 is biased in a distal direction by the spring member 554. Although shown in the form of a traditional coil spring, the spring member 554 may include any mechanism or material exhibiting elastic characteristics, e.g., pneumatic or hydraulic cylinder, rubber bumper. An elongate body portion 560 of the plunger member 552 is operably received within the longitudinal slot 319 formed in the extension 512 of the carrier 510. A distal end 560a of the elongate body portion 560 of the plunger member 552 engages the anvil member 522 of the anvil assembly 520 to bias the anvil assembly 520 to the open position (FIG. 40).

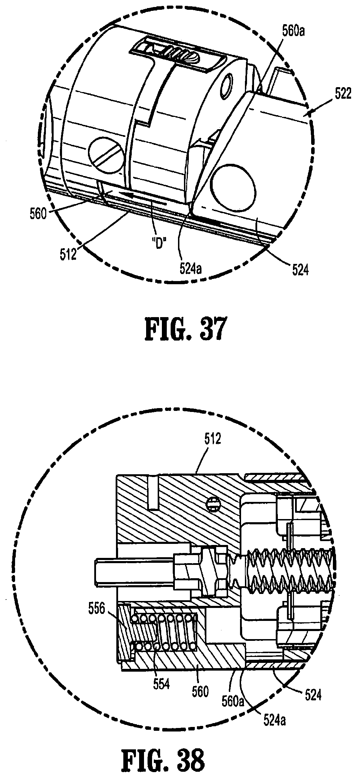

With reference now to FIGS. 37 and 38, as described above with regards to the loading unit 200, the loading unit 500 may be provided to the clinician in the closed position, the loading unit 500 may be closed prior to insertion into the body cavity (not shown) of a patient (not shown), or the loading unit 500 may be closed subsequent to positioning the tissue to be stapled (not shown) between the cartridge assembly 600 and the anvil member 522 of the anvil assembly 520. As the anvil member 522 is pivoted to the closed position, the proximal end 524a of the C-shaped frame 524 of the anvil member 522 engages the distal end 560a of the elongated body portion 560 of the plunger member 552 of the plunger assembly 550. The anvil assembly 520 may be moved to the closed position through advancement of the drive beam 540 of the actuation assembly 530 or through manual engagement of the anvil assembly 520 by the clinician. Engagement of the plunger member 552 of the plunger assembly 550 by the anvil member 522 as the anvil member 522 of the anvil assembly 520 is pivoted to the closed position causes the plunger member 552 to move in the proximal direction, as indicated by arrow "D". As plunger member 552 is moved proximally, i.e., retracted, spring member 554 of the plunger assembly 550 is compressed to provide a spring bias against anvil member 522.

Turning now to FIGS. 39 and 40, whether the anvil assembly 520 was held closed manually by a clinician or closed through operation of the drive beam 540 of the actuation assembly 530, release of the anvil assembly 520 by the clinician or disengagement of the drive beam 540 the anvil member 522 permits the anvil assembly 520 to move to the open position. The spring bias generated by compressing spring member 554 during the closing of anvil assembly 520 acts on plunger member 552 to move the plunger member 552 in the distal direction, as indicated by arrow "E". Engagement of the distal end 560a of the elongated body portion 560 of the plunger member 552 with the proximal end 524a of the C-shaped frame 524 of the anvil member 522 to cause the pivoting of the anvil member 522 to the open position.

Upon completion of a stapling procedure, the loading unit 500 may be separated or unloaded from the adapter assembly 400 in the manner described above. Additional loading units may then be attached to the adapter assembly 400 for subsequent stapling procedures. As noted above, the loading unit 500 may be configured to receive a replaceable cartridge assembly (not shown) for permitting reuse of the loading unit 500. The adapter assembly 400 may be separated from the handle assembly 10 (FIG. 1) and discarded. Alternatively, the adapter assembly 400 may be sterilized and reused.

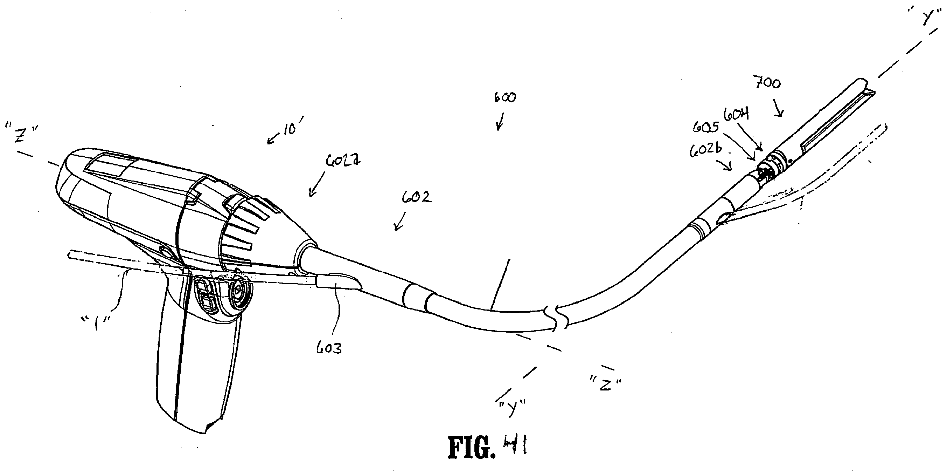

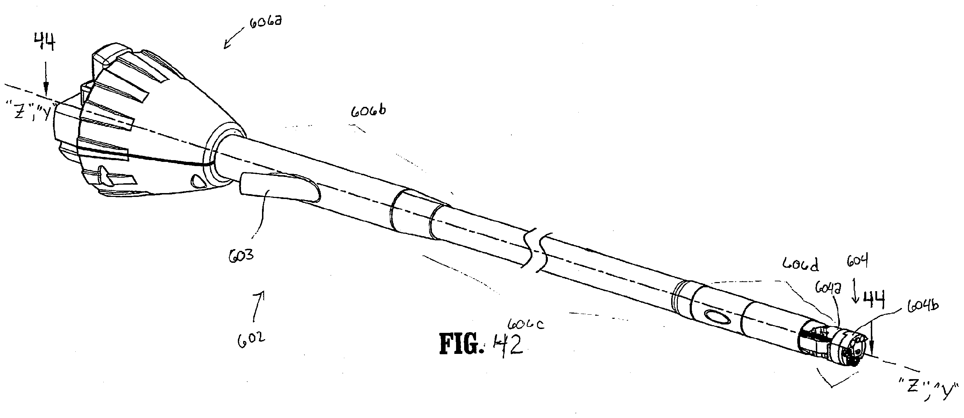

With reference now to FIGS. 41-46, an adapter assembly in accordance with an embodiment according to the invention is shown generally as adapter assembly 600. The adapter assembly 600 is similar to the adapter assembly 100 described hereinabove, and will therefore only be described in detail as relates to the differences therebetween.

With initial reference to FIGS. 41-43, the adapter assembly 600 includes a proximal body portion 602 and a distal body portion 604. The proximal body portion 602 includes a proximal end 602a configured for operable connection to a handle assembly 10' (FIG. 41) and a distal end 602b pivotally connected to a proximal end 604a of the distal body portion 604 at an articulation joint 605. The distal body portion 604 includes a distal end 604b configured for selective connection to a loading unit, e.g., a loading unit 700 (FIG. 41). Similar to the distal body portion 104 (FIG. 3) of the adapter assembly 100 (FIG. 3), the compact length of the distal body portion 604 of the adapter assembly 600 allows for greater manipulation of the loading unit 700 within a body cavity (not shown) during a surgical procedure. The ability of the distal body portion 604 to pivot through an arc of one-hundred eight degrees (180°) relative to the proximal body portion 602 allows for further manipulation of an attached loading unit (not shown).

With continued reference to FIGS. 41-43 the proximal body portion 602 of the adapter assembly 600 includes a handle portion 606a, a first elongate rigid portion 606b secured to and extending distally from the handle portion 606a, an elongate flexible portion 606c secured to and extending distally from the first elongate rigid portion 606b, and a second elongate rigid portion 606d secured to and extending from the elongate flexible portion 606c. The elongate flexible portion 606c may be of any length suitable for completing a given procedure. The flexibility of the elongate flexible portion 606c facilitates positioning of the loading unit 700 within body cavities (not shown) and through a lumen (not shown) of a patient (not shown), and increases the range of orientations in which the loading unit 700 may be positioned.

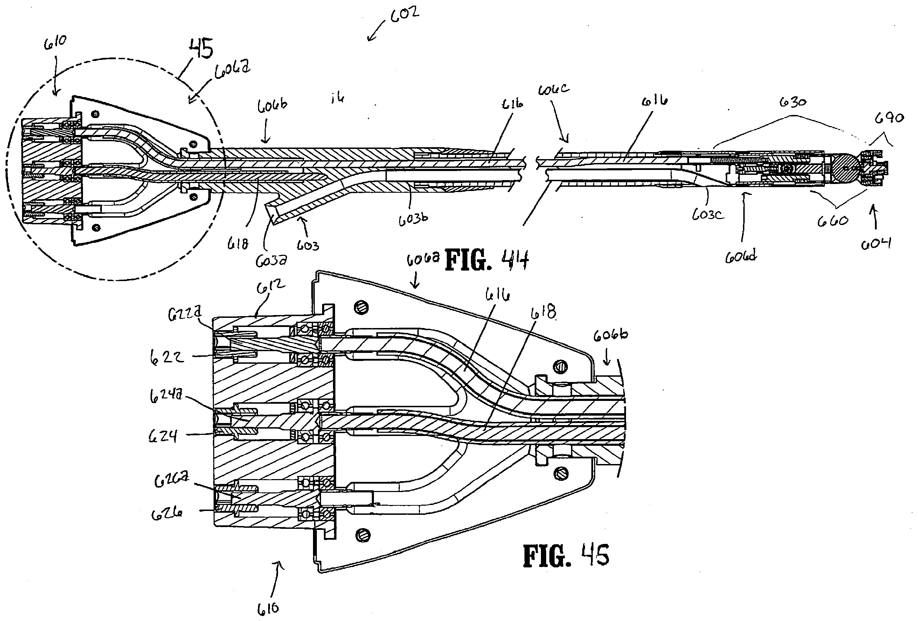

With particular reference now to FIGS. 44 and 45, a drive coupling assembly 610 extends proximally from the handle portion 606a of the proximal body portion 602 of the adapter assembly 600. An articulation assembly 630 (FIG. 44) is operably connected to the drive coupling assembly 610 and extends through the handle portions 606a, the first elongate rigid portion 606b, the elongate flexible portion 606c and the second elongate rigid portion 606d of the proximal body portion 602. A drive transfer assembly 660 (FIG. 44) extends between the proximal and distal body portions 602, 604 of the adapter assembly 600 and operably connects the drive coupling assembly 610 to a drive assembly 690 (FIG. 44) disposed within the distal body portion 604 of the adapter assembly 600. The drive coupling assembly 610, the articulation assembly 630, the drive transfer assembly 660, and the drive assembly 690 are substantially similar to the drive coupling assembly 110 (FIG. 10), the articulation assembly 130 (FIG. 10), the drive transfer assembly 160 (FIG. 15), and the drive assembly 190 (FIG. 15), respectively, described hereinabove, and will only be described to the extent necessary to fully disclose the adapter assembly 600.

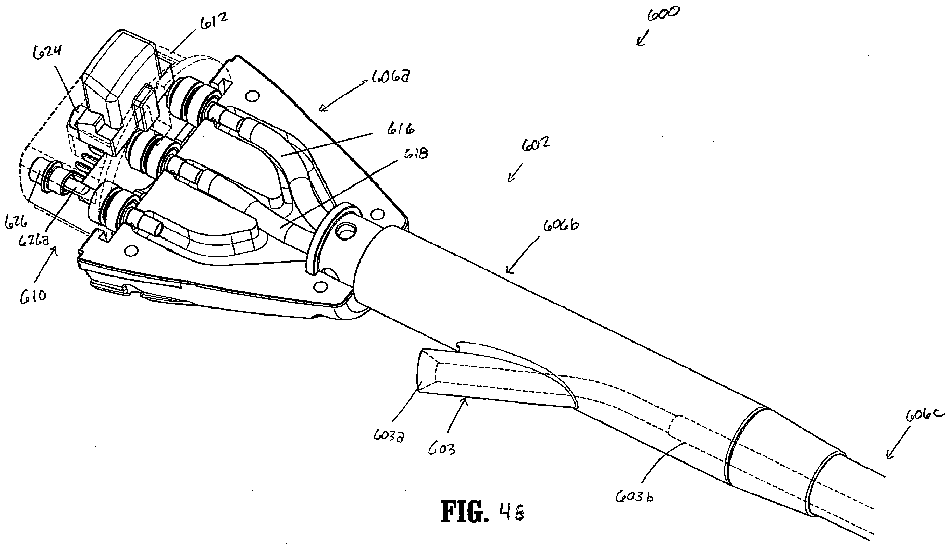

With additional reference to FIG. 46, the drive coupling assembly 610 includes a connector housing 612 for rotatably supporting first, second, and third connector sleeves 622, 624, and 626. Proximal ends of the first, second, and third connector sleeves 622, 624, and 626 are configured to mate with the respective first, second, and third drive members (not shown) of the handle assembly 10' (FIG. 41). Distal ends of the first, second, and third connector sleeves 622, 624, and 626 mate with first, second, and third connector shafts 622a, 624a, 626a. A distal end of the first connector shaft 622a engages a first drive shaft assembly 616, a distal end of the second connector shaft 624a engages a second drive shaft assembly 618, and a distal end of the third connector shaft 626a is configured to engage a third drive shaft assembly (not shown).

The first drive shaft assembly 616 operably connects the first drive member (not shown) of the actuation assembly 10' (FIG. 41) with the articulation assembly 630 (FIG. 44) for articulating the distal body portion 604 relative to the proximal body portion 602. The second drive shaft 618 operably connects the second drive member (not shown) of the actuation assembly 10' with the drive assembly 690 (FIG. 44) through the drive transfer assembly 660 (FIG. 44) to effect actuation of the loading unit 700 (FIG. 41). The third drive shaft (not shown) may operably connect the third drive member (not shown) of the actuation assembly 10' to the drive assembly 690 (FIG. 44) through the drive transfer assembly 660 to effect rotation of the loading unit 700 about its longitudinal axis "y".

With continued reference to FIG. 44, at least the portion of each of the first, second, and third drive shaft assemblies 616, 618, (not shown) that is disposed within the elongate flexible portion 606c of the proximal body portion 602 of the adapter assembly 600 is flexible. It is envisioned that any or all of the first, second, and third drive shaft assemblies 616, 618, (not shown) may be flexible in their entirety.

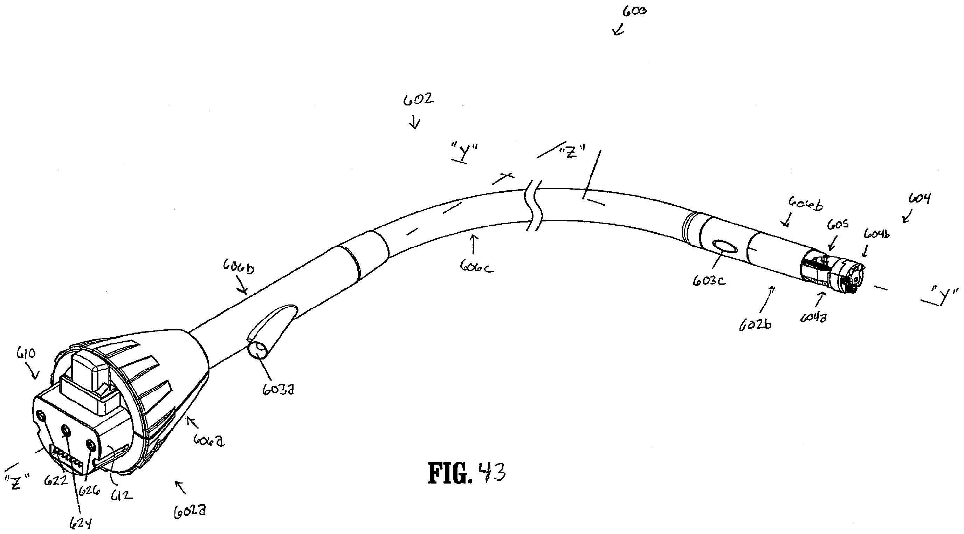

As shown, the handle portion 606a of the proximal body portion 602 of the adapter assembly 600 includes a frustoconical body configured for operable engagement by a user. Although not shown, it is envisioned that the adapter assembly 600 may be modified to permit rotation of the handle portion 606a relative to the drive coupling assembly 610. In this manner, the handle assembly 606a may be rotated relative to the actuation assembly 10' along a longitudinal axis "z" (FIG. 43) of the adapter assembly 600 to cause the loading unit 700, which defines a longitudinal axis "y" (FIG. 43), to articulate or pivot with respect to the longitudinal axis "z" of the adapter assembly 600 to permit positioning of the loading unit 700 relative to the actuation assembly 10'.

With particular reference to FIGS. 44 and 46, the first elongate rigid portion 606b of the proximal body portion 602 of the adapter assembly 600 includes a port 603. The port 603 defines a proximal opening 603a to a working channel or lumen 603b that extends from the first elongate rigid portion 606b of the proximal body portion 602, through the flexible elongate portion 606c of the proximal body portion 602, to a distal opening 603c defined in the second elongate rigid portion 606d of the proximal body portion 602. The working channel 603b provides a passageway for receipt of one or more instruments "I" (FIG. 41) through the adapter assembly 600 to a location proximal the loading unit 700 (FIG. 41). The working channel 603b may also be used for providing irrigation fluids to a surgical site and/or for aspirating fluids from the surgical site. Alternatively, or in addition, the working channel 603b may receive a guidewire (not shown) for directing the loading unit to a surgical site (not shown). It is envisioned that the working channel 603b may include one or more seal members to maintain a seal about an instrument "I" received therethrough. Although shown including only a single port 603, it is envisioned that the adapter assembly 600 may include more than one port and may define more than one working channel 603b.

In operation, the elongate flexible portion 606c of the adapter assembly 606 facilitates positioning of the loading unit 700 (FIG. 41) by permitting the adapter assembly 600 to flex along the longitudinal axis "z" to orient the loading 700 relative to the actuation assembly 10' (FIG. 41). As noted above, an instrument "I", a guidewire (not shown), irrigation fluid (not shown), and/or suction may be provided through the working channel 603b of the proximal body portion 602 of the adapter assembly 600 to a location proximal the loading unit 700 at any point during a surgical procedure. Once positioned the loading unit 700 is positioned about tissue to be staple (not shown), the adapter assembly 600 and the loading unit 700 operate in a similar manner to adapter assembly 100 and loading unit 200 described hereinabove.

Any of the components described herein may be fabricated from either metals, plastics, resins, composites or the like taking into consideration strength, durability, wearability, weight, resistance to corrosion, ease of manufacturing, cost of manufacturing, and the like.

## Claims

1. An adapter assembly (600) for selectively interconnecting a surgical end effector (700) that is configured to perform a function and a surgical device (10') that is configured to actuate the end effector, the end effector including at least one axially translatable drive member (240), and the surgical device including at least one rotatable drive shaft, the adapter assembly comprising:
a proximal body portion (602) configured for operable connection to the surgical device;
an articulation joint (605);
a distal body portion (604) selectively articulable relative to the proximal body portion at the articulation joint, the distal body portion including a distal end (604b) configured for selective engagement with the end effector and defining a longitudinal axis, wherein the proximal body portion includes an elongate flexible portion (606c) configured to orient the end effector relative to the surgical device,
wherein the proximal body portion defines a working channel (603b) therethrough,
**characterized in that** the working channel includes a proximal opening (603a) adjacent a proximal end (602a) of the proximal body portion and a distal opening (603b) adjacent a distal end (602b) of the proximal body portion.

2. The adapter assembly (600) of claim 1, wherein the length of the distal body portion (604) is substantially the same as the diameter of the distal body portion.

3. The adapter assembly (600) of claim 1 or claim 2, wherein the proximal body portion (602) further includes a handle portion (606a).

4. The adapter assembly (600) of claim 3, wherein the proximal body portion (602) further includes a first elongate rigid portion (606b) disposed between the handle portion (606a) and the elongate flexible portion (606c).

5. The adapter assembly (600) of claim 4, wherein the proximal body portion (602) further includes a second elongate rigid portion (606d) extending distally from the elongate flexible portion (606c).

6. The adapter assembly (600) of claim 3, wherein the handle portion (606a) includes a frustoconical body.

7. The adapter assembly (600) of claim 5, wherein the proximal opening (603a) of the working channel (603b) is defined in the first elongate rigid portion (606b), and the distal opening (603c) of the working channel is defined in the second elongate rigid portion (606d).

## Patentansprüche

1. Adapteranordnung (600) zum selektiven Verbinden eines chirurgischen Endeffektors (700), der konfiguriert ist, um eine Funktion auszuführen, und einer chirurgischen Vorrichtung (10'), die konfiguriert ist, um den Endeffektor zu betätigen, wobei der Endeffektor wenigstens ein axial verschiebbares Antriebselement (240) einschließt und die chirurgische Vorrichtung wenigstens eine drehbare Antriebswelle einschließt, wobei die Adapteranordnung Folgendes umfasst:
einen proximalen Körperabschnitt (602), der für eine Wirkverbindung mit der chirurgischen Vorrichtung konfiguriert ist;
eine Gelenkverbindung (605);
einen distalen Körperabschnitt (604), der selektiv relativ zu dem proximalen Körperabschnitt an der Gelenkverbindung gelenkig lagerbar ist, wobei der distale Körperabschnitt ein distales Ende (604b) einschließt, das zum selektiven Eingriff mit dem Endeffektor konfiguriert ist und eine Längsachse definiert, wobei der proximale Körperabschnitt einen länglichen flexiblen Abschnitt (606c) einschließt, der konfiguriert ist, um den Endeffektor relativ zu der chirurgischen Vorrichtung auszurichten,
wobei der proximale Körperabschnitt einen Arbeitskanal (603b) durch diesen definiert,
**dadurch gekennzeichnet, dass** der Arbeitskanal eine proximale Öffnung (603a) angrenzend an ein proximales Ende (602a) des proximalen Körperabschnitts und eine distale Öffnung (603b) angrenzend an ein distales Ende (602b) des proximalen Körperabschnitts einschließt.

2. Adapteranordnung (600) nach Anspruch 1, wobei die Länge des distalen Körperabschnitts (604) im Wesentlichen gleich dem Durchmesser des distalen Körperabschnitts ist.

3. Adapteranordnung (600) nach Anspruch 1 oder 2, wobei der proximale Körperabschnitt (602) ferner einen Griffabschnitt (606a) einschließt.

4. Adapteranordnung (600) nach Anspruch 3, wobei der proximale Körperabschnitt (602) ferner einen ersten länglichen starren Abschnitt (606b) einschließt, der zwischen dem Griffabschnitt (606a) und dem länglichen flexiblen Abschnitt (606c) angeordnet ist.

5. Adapteranordnung (600) nach Anspruch 4, wobei der proximale Körperabschnitt (602) ferner einen zweiten länglichen starren Abschnitt (606d) einschließt, der sich distal von dem länglichen flexiblen Abschnitt (606c) erstreckt.

6. Adapteranordnung (600) nach Anspruch 3, wobei der Griffabschnitt (606a) einen kegelstumpfförmigen Körper einschließt.

7. Adapteranordnung (600) nach Anspruch 5, wobei die proximale Öffnung (603a) des Arbeitskanals (603b) in dem ersten länglichen starren Abschnitt (606b) definiert ist und die distale Öffnung (603c) des Arbeitskanals in dem zweiten länglichen starren Abschnitt (606d) definiert ist.

## Revendications

1. Ensemble adaptateur (600) pour l'interconnexion sélective d'un effecteur terminal chirurgical (700) conçu pour exécuter une fonction et d'un dispositif chirurgical (10') conçu pour actionner l'effecteur terminal, l'effecteur terminal comportant au moins un élément d'entraînement (240) pouvant être translaté axialement, et le dispositif chirurgical comportant au moins un arbre d'entraînement rotatif, l'ensemble adaptateur comprenant :
une partie de corps proximale (602) conçue pour une liaison fonctionnelle au dispositif chirurgical ;
une charnière articulée (605) ;
une partie de corps distale (604) articulable sélectivement par rapport à la partie de corps proximale au niveau de la charnière articulée, la partie de corps distale comportant une extrémité distale (604b) conçue pour une mise en prise sélective avec l'effecteur terminal et définissant un axe longitudinal, dans lequel la partie de corps proximale comporte une partie flexible allongée (606c) conçue pour orienter l'effecteur terminal par rapport au dispositif chirurgical,
dans lequel la partie de corps proximale définit un canal de travail (603b) à travers celle-ci,
**caractérisé en ce que** le canal de travail comporte une ouverture proximale (603a) adjacente à une extrémité proximale (602a) de la partie de corps proximale et une ouverture distale (603b) adjacente à une extrémité distale (602b) de la partie de corps proximale.

2. Ensemble adaptateur (600) selon la revendication 1, dans lequel la longueur de la partie de corps distale (604) est sensiblement la même que le diamètre de la partie de corps distale.

3. Ensemble adaptateur (600) selon la revendication 1 ou la revendication 2, dans lequel la partie de corps proximale (602) comporte en outre une partie de poignée (606a).

4. Ensemble adaptateur (600) selon la revendication 3, dans lequel la partie corps proximale (602) comporte en outre une première partie rigide allongée (606b) disposée entre la partie poignée (606a) et la partie flexible allongée (606c).

5. Ensemble adaptateur (600) selon la revendication 4, dans lequel la partie de corps proximale (602) comporte en outre une seconde partie rigide allongée (606d) s'étendant distalement à partir de la partie flexible allongée (606c).

6. Ensemble adaptateur (600) selon la revendication 3, dans lequel la partie poignée (606a) comporte un corps tronconique.

7. Ensemble adaptateur (600) selon la revendication 5, dans lequel l'ouverture proximale (603a) du canal de travail (603b) est définie dans la première partie rigide allongée (606b), et l'ouverture distale (603c) du canal de travail est définie dans la seconde partie rigide allongée (606d) .
